# EUROPEAN PATENT SPECIFICATION

(11) **EP 2 618 761 B1**
(45) Date of publication and mention of the grant of the patent: **28.03.2018**
(21) Application number: 11764433.6
(22) Date of filing: 22.09.2011
(51) Int. Cl.: A61B 17/072, A61B 17/00, A61B 17/29, A61B 18/14, A61B 17/295

(54) **CONTROL FEATURES FOR ARTICULATING SURGICAL DEVICE**
STEUERFUNKTIONEN FÜR EINE GELENKIGE CHIRURGISCHE VORRICHTUNG
ÉLÉMENTS DE COMMANDE SERVANT À ARTICULER UN DISPOSITIF CHIRURGICAL

(30) Priority: 24.09.2010 US 386094 P
(43) Date of publication of application: 31.07.2013
(73) Proprietor: Ethicon LLC, 00969 Guaynabo (PR)
(72) Inventor: WORRELL, Barry, C., Centerville OH 45458 (US); BOURDREAUX, Chad, B., Cincinnati OH 45242 (US); MILLER, Matthew, C., Cincinnati, OH 45212 (US); SCHEIB, Charles, J., Loveland OH 45140 (US); SHELTON, Frederick, E., IV, Hillsboro, OH 45133 (US); STROBL, Geoffrey, S., Williamsburg OH 45176 (US); STULEN, Foster, B., Mason OH 45040 (US); TREES, Gregory, A., Loveland OH 45140 (US); VOEGELE, Aaron, C., Loveland OH 45140 (US); MADSEN, Steven, D., New York NY 10026 (US); HENRY, Emron, Cincinnati, OH 45241 (US); CLEM, William, E., Bozeman MT 59715 (US)
(74) Representative: Tunstall, Christopher Stephen
(86) International application number: PCT/US2011/052712
(87) International publication number: WO 2012/040432

(56) References cited:
- EP-A1- 2 151 204
- US-A- 5 704 534
- US-A1- 2009 198 272
- US-A1- 2010 094 289

## Description

### BACKGROUND

A variety of surgical instruments include a tissue cutting element and one or more elements that transmit RF energy to tissue (e.g., to coagulate or seal the tissue). An example of such a device is the ENSEAL® Tissue Sealing Device by Ethicon Endo-Surgery, Inc., of Cincinnati, Ohio. Further examples of such devices and related concepts are disclosed in U.S. Pat. No. 6,500,176 entitled "Electrosurgical Systems and Techniques for Sealing Tissue," issued December 31, 2002, U.S. Pat. No. 7,112,201 entitled "Electrosurgical Instrument and Method of Use," issued September 26, 2006, U.S. Pat. No. 7,125,409, entitled "Electrosurgical Working End for Controlled Energy Delivery," issued October 24, 2006, U.S. Pat. No. 7,169,146 entitled "Electrosurgical Probe and Method of Use," issued January 30, 2007, U.S. Pat. No. 7,186,253, entitled "Electrosurgical Jaw Structure for Controlled Energy Delivery," issued March 6, 2007, U.S. Pat. No. 7,189,233, entitled "Electrosurgical Instrument," issued March 13, 2007, U.S. Pat. No. 7,220,951, entitled "Surgical Sealing Surfaces and Methods of Use," issued May 22, 2007, U.S. Pat. No. 7,309,849, entitled "Polymer Compositions Exhibiting a PTC Property and Methods of Fabrication," issued December 18, 2007, U.S. Pat. No. 7,311,709, entitled "Electrosurgical Instrument and Method of Use," issued December 25, 2007, U.S. Pat. No. 7,354,440, entitled "Electrosurgical Instrument and Method of Use," issued April 8, 2008, U.S. Pat. No. 7,381,209, entitled "Electrosurgical Instrument," issued June 3, 2008, U.S. Pub. No. 2011/0087218, entitled "Surgical Instrument Comprising First and Second Drive Systems Actuatable by a Common Trigger Mechanism," published April 14, 2011, and U.S. Pat. App. No. 13/151,181, entitled "Motor Driven Electrosurgical Device with Mechanical and Electrical Feedback," filed June 2, 2011.

In addition, a variety of surgical instruments include a shaft having an articulating section, providing enhanced positioning capabilities for an end effector that is located distal to the articulating section of the shaft. Such devices are known for example from US 2009/0198272 A1, US 2010/0094289 A1 or EP 2 151 204 A1. Further examples of such devices include various models of the ENDOPATH® endocutters by Ethicon Endo-Surgery, Inc., of Cincinnati, Ohio. Further examples of such devices and related concepts are disclosed in U.S. Pat. No. 7,380,696, entitled "Articulating Surgical Stapling Instrument Incorporating a Two-Piece E-Beam Firing Mechanism," issued June 3, 2008, U.S. Pat. No. 7,404,508, entitled "Surgical Stapling and Cutting Device," issued July 29, 2008, reference herein; U.S. Pat. No. 7,455,208, entitled "Surgical Instrument with Articulating Shaft with Rigid Firing Bar Supports," issued November 25, 2008, U.S. Pat. No. 7,506,790, entitled "Surgical Instrument Incorporating an Electrically Actuated Articulation Mechanism," issued March 24, 2009, U.S. Pat. No. 7,549,564, entitled "Surgical Stapling Instrument with an Articulating End Effector," issued June 23, 2009, U.S. Pat. No. 7,559,450, entitled "Surgical Instrument Incorporating a Fluid Transfer Controlled Articulation Mechanism," issued July 14, 2009, U.S. Pat. No. 7,654,431, entitled "Surgical Instrument with Guided Laterally Moving Articulation Member," issued February 2, 2010, U.S. Pat. No. 7,780,054, entitled "Surgical Instrument with Laterally Moved Shaft Actuator Coupled to Pivoting Articulation Joint," issued August 24, 2010, U.S. Pat. No. 7,784,662, entitled "Surgical Instrument with Articulating Shaft with Single Pivot Closure and Double Pivot Frame Ground," issued August 31, 2010, and U.S. Pat. No. 7,798,386, entitled "Surgical Instrument Articulation Joint Cover," issued September 21, 2010.

While several medical devices have been made and used, it is believed that no one prior to the inventors has made or used the invention described in the appended claims. The invention is defined in claim 1. Further aspects and preferred embodiments are defined in the dependent claims. Aspects, embodiments and examples of the present disclosure which do not fall under the scope of the appended claims do not form part of the invention and are merely provided for illustrative purposes.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a side elevational view of an exemplary electrosurgical medical device;
FIG. 2 depicts a perspective view of the end effector of the device of FIG. 1, in an open configuration;
FIG. 3 depicts another perspective view of the end effector of the device of FIG. 1, in an open configuration;
FIG. 4 depicts a cross-sectional end view of the end effector of FIG. 2, in a closed configuration and with the blade in a distal position;
FIG. 5A depicts an end view of an electrosurgical instrument according to the present invention with a laterally pivoting trigger in a home position and a plan view of the end effector of the instrument in a non-articulated position;
FIG. 5B depicts an end view of the electrosurgical instrument of FIG. 5A and a plan view of the end effector of FIG. 5A, with the laterally pivoting trigger in a first pivoted position and with the end effector in a first articulated position;
FIG. 5C depicts an end view of the electrosurgical instrument of FIG. 5A and a plan view of the end effector of FIG. 5A, with the laterally pivoting trigger in a second pivoted position and with the end effector in a second articulated position;
FIG. 6 depicts a partial perspective view of an articulation section and end effector of another exemplary electrosurgical instrument;
FIG. 7 depicts a perspective cross-sectional view of the articulation section of FIG. 6;
FIG. 8 depicts a perspective view of articulation control features and a firing beam extending to the end effector of FIG. 6;
FIG. 9 depicts a perspective view of a proximal portion of the articulation control features of FIG. 8, with camming features omitted;
FIG. 10 depicts a perspective view of the proximal portion of FIG. 9, with camming features included;
FIG. 11 depicts a perspective view of the proximal portion and camming features of FIG. 10, with half of a drive nut included;
FIG. 12 depicts a side elevational view of the drive nut half of FIG. 11;
FIG. 13A depicts a partial perspective view of the articulation control features of FIG. 11 disposed in a handle, with a rotator in a distal position;
FIG. 13B depicts a partial perspective view of the components of FIG. 13A, with the rotator in a proximal position;
FIG. 14A depicts another partial perspective view of the components of FIG. 13A, with the rotator in partial cross-section and with the rotator in a distal position;
FIG. 14B depicts another partial perspective view of the components of FIG. 13A, with the rotator in partial cross-section and with the rotator in a proximal position;
FIG. 15 depicts a perspective view of half of the rotator of FIG. 13A;
FIG. 16 depicts a perspective view of another exemplary electrosurgical instrument;
FIG. 17 depicts a partial perspective view of the underside of articulation control components of the instrument of FIG. 16;
FIG. 18 depicts a plan view of the underside of the articulation control components of FIG. 17;
FIG. 19 depicts a partial cross-sectional view of the articulation control components of FIG. 17, taken along line 19-19 of FIG. 16;
FIG. 20 depicts a partial cross-sectional view of another electrosurgical instrument, showing exemplary alternative articulation control components;
FIG. 21 depicts an elevational view of a spool component of the articulation control components of FIG. 20;
FIG. 22 depicts a partial cross-sectional view of another electrosurgical instrument, showing another set of exemplary alternative articulation control components;
FIG. 23 depicts a partial perspective view of another electrosurgical instrument, showing an exemplary alternative placement for an articulation control feature;
FIG. 24 depicts a partial cross-sectional view of another electrosurgical instrument, showing another set of exemplary alternative articulation control components;
FIG. 25 depicts a partial cross-sectional view of another electrosurgical instrument, showing another set of exemplary alternative articulation control components;
FIG. 26 depicts a perspective view of another exemplary electrosurgical instrument, having a rotatable pistol grip;
FIG. 27 depicts a side elevational view of another exemplary electrosurgical instrument, having a pivoting top section;
FIG. 28A depicts a top plan view of the instrument of FIG. 27, with the top section in a home position and with the end effector in a home position;
FIG. 28B depicts a top plan view of the instrument of FIG. 27, with the top section in a pivoted position and with the end effector in an articulated position;
FIG. 29 depicts a side elevational view of another exemplary electrosurgical instrument, having a pivoting distal section;
FIG. 30 depicts a top plan view of the instrument of FIG. 29, with the distal section in a pivoted position;
FIG. 31 depicts a partial perspective view of another exemplary electrosurgical instrument, having a clutching articulation control;
FIG. 32 depicts a cross-sectional view of articulation control components of the instrument of FIG. 31, taken along line 32-32 of FIG. 31;
FIG. 33 depicts a partial perspective view of articulation components of the instrument of FIG. 31;
FIG. 34A depicts a cross-sectional end view and enlarged view of clutching features of the articulation components of FIG. 33, in an engaged configuration;
FIG. 34B depicts a cross-sectional end view and enlarged view of the clutching features of FIG. 34A, in a disengaged configuration;
FIG. 35 depicts a partial perspective view of another exemplary electrosurgical instrument, having an exemplary alternative clutching articulation control;
FIG. 36 depicts a side elevational view of another exemplary electrosurgical instrument, having a multi-mode trigger;
FIG. 37 depicts a side elevational view of another exemplary electrosurgical instrument, having an articulation trigger;
FIG. 38 depicts a perspective view of the instrument of FIG. 37, with the trigger pivoted to articulate an end effector;
FIG. 39 depicts a perspective view of another exemplary electrosurgical instrument, having an articulation control cam;
FIG. 40 depicts a partial perspective view of articulation control components of the instrument of FIG. 39;
FIG. 41 depicts a top plan view of the articulation control cam of FIG. 39;
FIG. 42 depicts a perspective view of the articulation control cam of FIG. 39; and
FIG. 43 depicts a perspective view of the articulation control cam of FIG. 39 engaged with other articulation control components.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

### I. Exemplary Electrosurgical Device with Articulation Feature

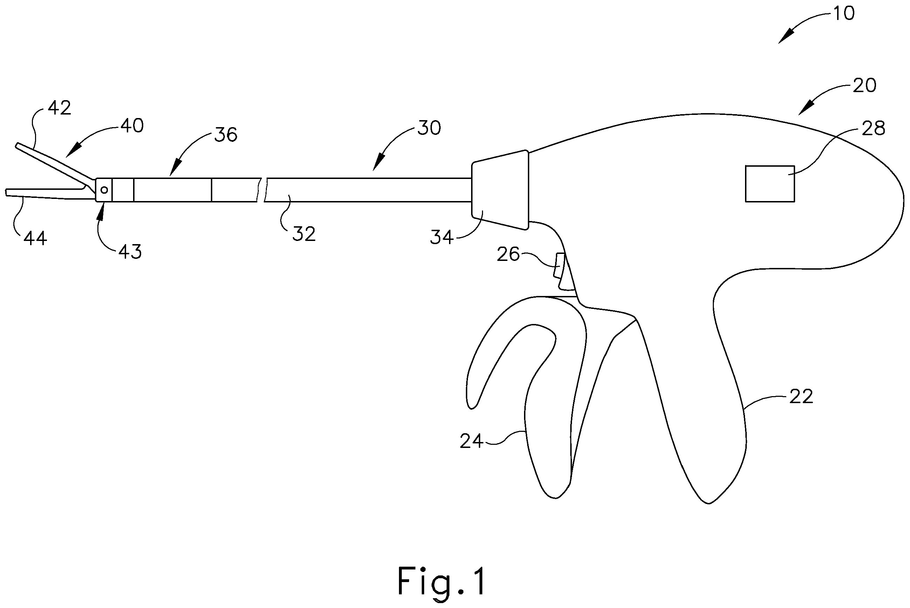

FIGS. 1-4 show an exemplary electrosurgical instrument (10) that is constructed and operable in accordance with at least some of the teachings of U.S. Pat. No. 6,500,176; U.S. Pat. No. 7,112,201; U.S. Pat. No. 7,125,409; U.S. Pat. No. 7,169,146; U.S. Pat. No. 7,186,253; U.S. Pat. No. 7,189,233; U.S. Pat. No. 7,220,951; U.S. Pat. No. 7,309,849; U.S. Pat. No. 7,311,709; U.S. Pat. No. 7,354,440; U.S. Pat. No. 7,381,209; U.S. Pub. No. 2011/0087218; and/or U.S. Pat. App. No. 13/151,181. As described therein and as will be described in greater detail below, electrosurgical instrument (10) is operable to cut tissue and seal or weld tissue (e.g., a blood vessel, etc.) substantially simultaneously. In other words, electrosurgical instrument (10) operates similar to an endocutter type of stapler, except that electrosurgical instrument (10) provides tissue welding through application of bipolar RF energy instead of providing lines of staples to join tissue. It should also be understood that electrosurgical instrument (10) may have various structural and functional similarities with the ENSEAL® Tissue Sealing Device by Ethicon Endo-Surgery, Inc., of Cincinnati, Ohio. Furthermore, electrosurgical instrument (10) may have various structural and functional similarities with the devices taught in any of the other references that are cited and incorporated by reference herein. To the extent that there is some degree of overlap between the teachings of the references cited herein, the ENSEAL® Tissue Sealing Device by Ethicon Endo-Surgery, Inc., of Cincinnati, Ohio, and the following teachings relating to electrosurgical instrument (10), there is no intent for any of the description herein to be presumed as admitted prior art. Several teachings below will in fact go beyond the scope of the teachings of the references cited herein and the ENSEAL® Tissue Sealing Device by Ethicon Endo-Surgery, Inc., of Cincinnati, Ohio.

### A. Exemplary Handpiece and Shaft

Electrosurgical instrument (10) of the present example includes a handpiece (20), a shaft (30) extending distally from handpiece (20), and an end effector (40) disposed at a distal end of shaft (30). Handpiece (20) of the present example includes a pistol grip (22), a pivoting trigger (24), an activation button (26), and an articulation control (28). Trigger (24) is pivotable toward and away from pistol grip (22) to selectively actuate end effector (40) as will be described in greater detail below. Activation button (26) is operable to selectively activate RF circuitry that is in communication with end effector (40), as will also be described in greater detail below. In some versions, activation button (26) also serves as a mechanical lockout against trigger (24), such that trigger (24) cannot be fully actuated unless button (26) is being pressed simultaneously. Examples of how such a lockout may be provided are disclosed in one or more of the references cited herein. It should be understood that pistol grip (22), trigger (24), and button (26) may be modified, substituted, supplemented, etc. in any suitable way, and that the descriptions of such components herein are merely illustrative. Articulation control (28) of the present example is operable to selectively control articulation section (36) of shaft (30), which will be described in greater detail below. Various examples of forms that articulation control (28) may take will also be described in greater detail below, while further examples will be apparent to those of ordinary skill in the art in view of the teachings herein.

Shaft (30) of the present example includes an outer sheath (32) and an articulating section (36). Articulating section (36) is operable to selectively position end effector (40) at various angles relative to the longitudinal axis defined by sheath (32). Various examples of forms that articulating section (36) and other components of shaft (30) may take will be described in greater detail below, while further examples will be apparent to those of ordinary skill in the art in view of the teachings herein. For instance, it should be understood that various components that are operable to actuate articulating section (36) may extend through the interior of sheath (32). In some versions, shaft (30) is also rotatable about the longitudinal axis defined by sheath (32), relative to handpiece (20), via a knob (34). Such rotation may provide rotation of end effector (40) and shaft (30) unitarily. In some other versions, knob (34) is operable to rotate end effector (40) without rotating any portion of shaft (30) that is proximal of articulating section (36). As another merely illustrative example, electrosurgical instrument (10) may include one rotation control that provides rotatability of shaft (30) and end effector (40) as a single unit; and another rotation control that provides rotatability of end effector (40) without rotating any portion of shaft (30) that is proximal of articulating section (36). Other suitable rotation schemes will be apparent to those of ordinary skill in the art in view of the teachings herein. Of course, rotatable features may simply be omitted if desired.

### B. Exemplary End Effector

End effector (40) of the present example comprises a first jaw (42) and a second jaw (44). In the present example, second jaw (44) is substantially fixed relative to shaft (30); while first jaw (42) pivots relative to shaft (30), toward and away from second jaw (42). In some versions, actuators such as rods or cables, etc., may extend through sheath (32) and be joined with first jaw (42) at a pivotal coupling (43), such that longitudinal movement of the actuator rods/cables/etc. through shaft (30) provides pivoting of first jaw (42) relative to shaft (30) and relative to second jaw (44). Of course, jaws (42, 44) may instead have any other suitable kind of movement and may be actuated in any other suitable fashion. By way of example only, and as will be described in greater detail below, jaws (42, 44) may be actuated and thus closed by longitudinal translation of a firing beam (60), such that actuator rods/cables/etc. may simply be eliminated in some versions.

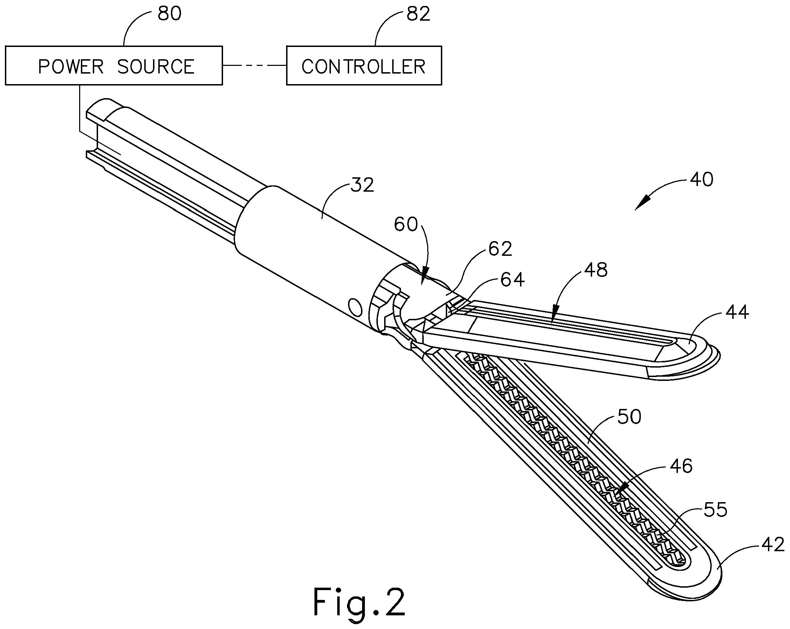

As best seen in FIGS. 2-4, first jaw (42) defines a longitudinally extending elongate slot (46); while second jaw (44) also defines a longitudinally extending elongate slot (48). In addition, the top side of first jaw (42) presents a first electrode surface (50); while the underside of second jaw (44) presents a second electrode surface (52). Electrode surfaces (50, 52) are in communication with an electrical source (80) via one or more conductors (not shown) that extend along the length of shaft (30). Electrical source (80) is operable to deliver RF energy to first electrode surface (50) at a first polarity and to second electrode surface (52) at a second (opposite) polarity, such that RF current flows between electrode surfaces (50, 52) and thereby through tissue captured between jaws (42, 44). In some versions, firing beam (60) serves as an electrical conductor that cooperates with electrode surfaces (50, 52) (e.g., as a ground return) for delivery of bipolar RF energy captured between jaws (42, 44). Electrical source (80) may be external to electrosurgical instrument (10) or may be integral with electrosurgical instrument (10) (e.g., in handpiece (20), etc.), as described in one or more references cited herein or otherwise. A controller (82) regulates delivery of power from electrical source (80) to electrode surfaces (50, 52). Controller (82) may also be external to electrosurgical instrument (10) or may be integral with electrosurgical instrument (10) (e.g., in handpiece (20), etc.), as described in one or more references cited herein or otherwise. It should also be understood that electrode surfaces (50, 52) may be provided in a variety of alternative locations, configurations, and relationships.

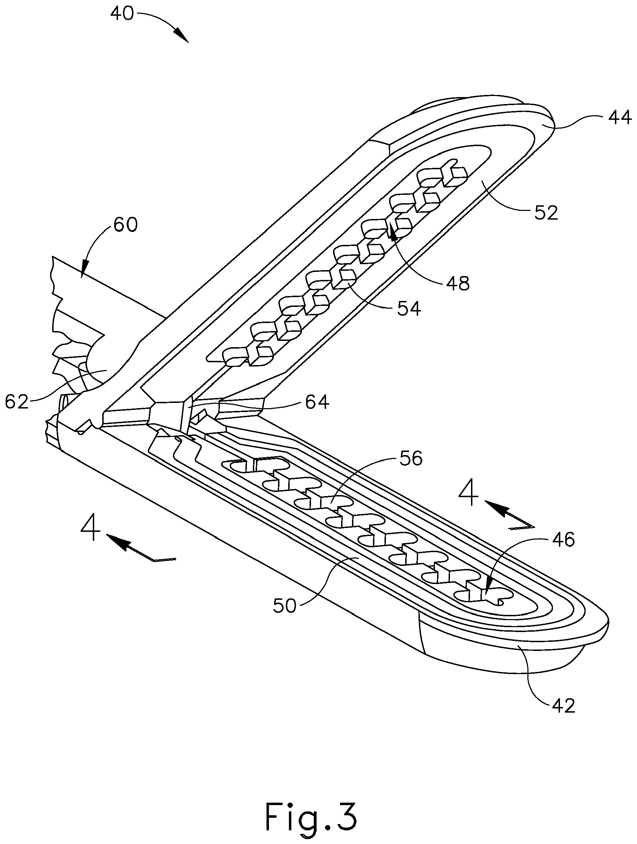

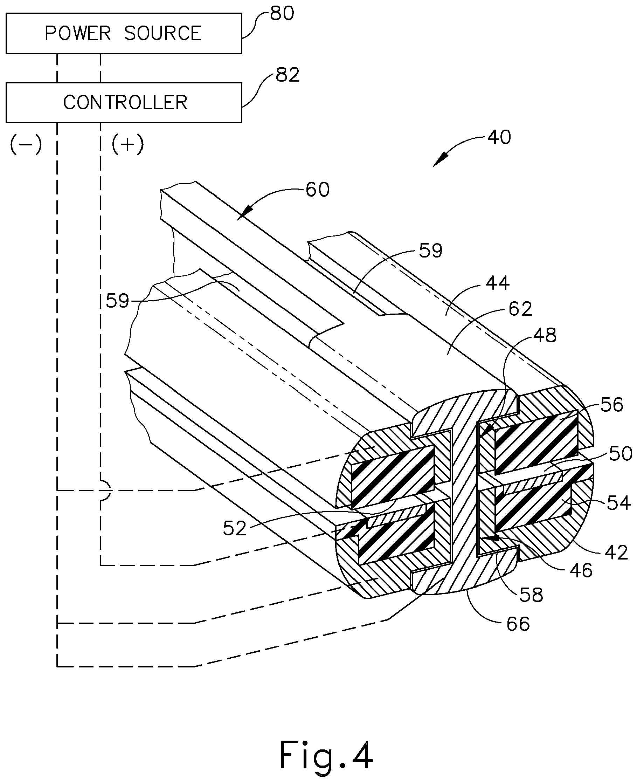

As best seen in FIG. 4, the lower side of first jaw (42) includes a longitudinally extending recess (58) adjacent to slot (46); while the upper side of second jaw (44) includes a longitudinally extending recess (58) adjacent to slot (48). FIG. 2 shows the upper side of first jaw (42) including a plurality of teeth serrations (46). It should be understood that the lower side of second jaw (44) may include complementary serrations that nest with serrations (46), to enhance gripping of tissue captured between jaws (42, 44) without necessarily tearing the tissue. FIG. 3 shows an example of serrations (46) in first jaw (42) as mainly recesses; with serrations (48) in second jaw (44) as mainly protrusions. Of course, serrations (46, 48) may take any other suitable form or may be simply omitted altogether. It should also be understood that serrations (46, 48) may be formed of an electrically non-conductive, or insulative, material, such as plastic, glass, and/or ceramic, for example, and may include a treatment such as polytetrafluoroethylene, a lubricant, or some other treatment to substantially prevent tissue from getting stuck to jaws (42, 44).

With jaws (42, 44) in a closed position, shaft (30) and end effector (40) are sized and configured to fit through trocars having various inner diameters, such that electrosurgical instrument (10) is usable in minimally invasive surgery, though of course electrosurgical instrument (10) could also be used in open procedures if desired. By way of example only, with jaws (42, 44) in a closed position, shaft (30) and end effector (40) may present an outer diameter of approximately 5 mm. Alternatively, shaft (30) and end effector (40) may present any other suitable outer diameter (e.g., between approximately 2 mm and approximately 20 mm, etc.).

As another merely illustrative variation, either jaw (42, 44) or both of jaws (42, 44) may include at least one port, passageway, conduit, and/or other feature that is operable to draw steam, smoke, and/or other gases/vapors/etc. from the surgical site. Such a feature may be in communication with a source of suction, such as an external source or a source within handpiece (20), etc. In addition, end effector (40) may include one or more tissue cooling features (not shown) that reduce the degree or extent of thermal spread caused by end effector (40) on adjacent tissue when electrode surfaces (50, 52) are activated. Various suitable forms that such cooling features may take will be apparent to those of ordinary skill in the art in view of the teachings herein.

In some versions, end effector (40) includes one or more sensors (not shown) that are configured to sense a variety of parameters at end effector (40), including but not limited to temperature of adjacent tissue, electrical resistance or impedance of adjacent tissue, voltage across adjacent tissue, forces exerted on jaws (42, 44) by adjacent tissue, etc. By way of example only, end effector (40) may include one or more positive temperature coefficient (PTC) thermistor bodies (54, 56) (e.g., PTC polymer, etc.), located adjacent to electrodes (50, 52) and/or elsewhere. Data from sensors may be communicated to controller (82). Controller (82) may process such data in a variety of ways. By way of example only, controller (82) may modulate or otherwise change the RF energy being delivered to electrode surfaces (50, 52), based at least in part on data acquired from one or more sensors at end effector (40). In addition or in the alternative, controller (82) may alert the user to one or more conditions via an audio and/or visual feedback device (e.g., speaker, lights, display screen, etc.), based at least in part on data acquired from one or more sensors at end effector (40). It should also be understood that some kinds of sensors need not necessarily be in communication with controller (82), and may simply provide a purely localized effect at end effector (40). For instance, a PTC thermistor bodies (54, 56) at end effector (40) may automatically reduce the energy delivery at electrode surfaces (50, 52) as the temperature of the tissue and/or end effector (40) increases, thereby reducing the likelihood of overheating. In some such versions, a PTC thermistor element is in series with power source (80) and electrode surface (50, 52); and the PTC thermistor provides an increased impedance (reducing flow of current) in response to temperatures exceeding a threshold. Furthermore, it should be understood that electrode surfaces (50, 52) may be used as sensors (e.g., to sense tissue impedance, etc.). Various kinds of sensors that may be incorporated into electrosurgical instrument (10) will be apparent to those of ordinary skill in the art in view of the teachings herein. Similarly various things that can be done with data from sensors, by controller (82) or otherwise, will be apparent to those of ordinary skill in the art in view of the teachings herein. Other suitable variations for end effector (40) will also be apparent to those of ordinary skill in the art in view of the teachings herein.

### C. Exemplary Firing Beam

As also seen in FIGS. 2-4, electrosurgical instrument (10) of the present example includes a firing beam (60) that is longitudinally movable along part of the length of end effector (40). Firing beam (60) is coaxially positioned within shaft (30), extends along the length of shaft (30), and translates longitudinally within shaft (30) (including articulating section (36) in the present example), though it should be understood that firing beam (60) and shaft (30) may have any other suitable relationship. Firing beam (60) includes a sharp distal blade (64), an upper flange (62), and a lower flange (66). As best seen in FIG. 4, distal blade (64) extends through slots (46, 48) of jaws (42, 44), with upper flange (62) being located above jaw (44) in recess (59) and lower flange (66) being located below jaw (42) in recess (58). The configuration of distal blade (64) and flanges (62, 66) provides an "I-beam" type of cross section at the distal end of firing beam (60). While flanges (62, 66) extend longitudinally only along a small portion of the length of firing beam (60) in the present example, it should be understood that flanges (62, 66) may extend longitudinally along any suitable length of firing beam (60). In addition, while flanges (62, 66) are positioned along the exterior of jaws (42, 44), flanges (62, 66) may alternatively be disposed in corresponding slots formed within jaws (42, 44). For instance, each jaw (42, 44) may define a "T"-shaped slot, with parts of distal blade (64) being disposed in one vertical portion of each "T"-shaped slot and with flanges (62, 66) being disposed in the horizontal portions of the "T"-shaped slots. Various other suitable configurations and relationships will be apparent to those of ordinary skill in the art in view of the teachings herein.

Distal blade (64) is substantially sharp, such that distal blade will readily sever tissue that is captured between jaws (42, 44). Distal blade (64) is also electrically grounded in the present example, providing a return path for RF energy as described elsewhere herein. In some other versions, distal blade (64) serves as an active electrode. In addition or in the alternative, distal blade (64) may be selectively energized with ultrasonic energy (e.g., harmonic vibrations at approximately 55.5 kHz, etc.).

The "I-beam" type of configuration of firing beam (60) provides closure of jaws (42, 44) as firing beam (60) is advanced distally. In particular, flange (62) urges jaw (44) pivotally toward jaw (42) as firing beam (60) is advanced from a proximal position (FIGS. 1-3) to a distal position (FIG. 4), by bearing against recess (59) formed in jaw (44). This closing effect on jaws (42, 44) by firing beam (60) may occur before distal blade (64) reaches tissue captured between jaws (42, 44). Such staging of encounters by firing beam (60) may reduce the force required to squeeze grip (24) to actuate firing beam (60) through a full firing stroke. In other words, in some such versions, firing beam (60) may have already overcome an initial resistance required to substantially close jaws (42, 44) on tissue before encountering resistance from the tissue captured between jaws (42, 44). Of course, any other suitable staging may be provided.

In the present example, flange (62) is configured to cam against a ramp feature at the proximal end of jaw (44) to open jaw (42) when firing beam (60) is retracted to a proximal position and to hold jaw (42) open when firing beam (60) remains at the proximal position. This camming capability may facilitate use of end effector (40) to separate layers of tissue, to perform blunt dissections, etc., by forcing jaws (42, 44) apart from a closed position. In some other versions, jaws (42, 44) are resiliently biased to an open position by a spring or other type of resilient feature. While jaws (42, 44) close or open as firing beam (60) is translated in the present example, it should be understood that other versions may provide independent movement of jaws (42, 44) and firing beam (60). By way of example only, one or more cables, rods, beams, or other features may extend through shaft (30) to selectively actuate jaws (42, 44) independently of firing beam (60). Such jaw (42, 44) actuation features may be separately controlled by a dedicated feature of handpiece (20). Alternatively, such jaw actuation features may be controlled by trigger (24) in addition to having trigger (24) control firing beam (60). It should also be understood that firing beam (60) may be resiliently biased to a proximal position, such that firing beam (60) retracts proximally when a user relaxes their grip on trigger (24).

### D. Exemplary Operation

In an exemplary use, end effector (40) is inserted into a patient via a trocar. Articulation section (36) is substantially straight when end effector (40) and part of shaft (30) are inserted through the trocar. Articulation control (28) may then be manipulated to pivot or flex articulation section (36) of shaft (30) in order to position end effector (40) at a desired position and orientation relative to an anatomical structure within the patient. Two layers of tissue of the anatomical structure are then captured between jaws (42, 44) by squeezing trigger (24) toward pistol grip (22). Such layers of tissue may be part of the same natural lumen defining anatomical structure (e.g., blood veseel, portion of gastrointestinal tract, portion of reproductive system, etc.) in a patient. For instance, one tissue layer may comprise the top portion of a blood vessel while the other tissue layer may comprise the bottom portion of the blood vessel, along the same region of length of the blood vessel (e.g., such that the fluid path through the blood vessel before use of electrosurgical instrument (10) is perpendicular to the longitudinal axis defined by end effector (40), etc.). In other words, the lengths of jaws (42, 44) may be oriented perpendicular to (or at least generally transverse to) the length of the blood vessel. As noted above, flanges (62, 66) cammingly act to pivot jaw (44) toward jaw (44) when firing beam (60) is actuated distally by squeezing trigger (24) toward pistol grip (22).

With tissue layers captured between jaws (42, 44) firing beam (60) continues to advance distally by the user squeezing trigger (24) toward pistol grip (22). As firing beam (60) advances distally, distal blade (64) simultaneously severs the clamped tissue layers, resulting in separated upper layer portions being apposed with respective separated lower layer portions. In some versions, this results in a blood vessel being cut in a direction that is generally transverse to the length of the blood vessel. It should be understood that the presence of flanges (62, 66) immediately above and below jaws (42, 44), respectively, may help keep jaws (42, 44) in a closed and tightly clamping position. In particular, flanges (62, 66) may help maintain a significantly compressive force between jaws (42, 44). With severed tissue layer portions being compressed between jaws (42, 44), electrode surfaces (50, 52) are activated with bipolar RF energy by the user depressing activation button (26). In some versions, electrodes (50, 52) are selectively coupled with power source (80) (e.g., by the user depressing button (26), etc.) such that electrode surfaces (50, 52) of jaws (42, 44) are activated with a common first polarity while firing beam (60) is activated at a second polarity that is opposite to the first polarity. Thus, a bipolar RF current flows between firing beam (60) and electrode surfaces (50, 52) of jaws (42, 44), through the compressed regions of severed tissue layer portions. In some other versions, electrode surface (50) has one polarity while electrode surface (52) and firing beam (60) both have the other polarity. In either version (among at least some others), bipolar RF energy delivered by power source (80) ultimately thermally welds the tissue layer portions on one side of firing beam (60) together and the tissue layer portions on the other side of firing beam (60) together.

In certain circumstances, the heat generated by activated electrode surfaces (50, 52) can denature the collagen within the tissue layer portions and, in cooperation with clamping pressure provided by jaws (42, 44), the denatured collagen can form a seal within the tissue layer portions. Thus, the severed ends of the natural lumen defining anatomical structure are hemostatically sealed shut, such that the severed ends will not leak bodily fluids. In some versions, electrode surfaces (50, 52) may be activated with bipolar RF energy before firing beam (60) even begins to translate distally and thus before the tissue is even severed. For instance, such timing may be provided in versions where button (26) serves as a mechanical lockout relative to trigger (24) in addition to serving as a switch between power source (80) and electrode surfaces (50, 52).

While several of the teachings below are described as variations to electrosurgical instrument (10), it should be understood that various teachings below may also be incorporated into various other types of devices. By way of example only, in addition to being readily incorporated into electrosurgical instrument (10), various teachings below may be readily incorporated into the devices taught in any of the references cited herein, other types of electrosurgical devices, surgical staplers, surgical clip appliers, and tissue graspers, among various other devices. Other suitable devices into which the following teachings may be incorporated will be apparent to those of ordinary skill in the art in view of the teachings herein.

### II. Exemplary Articulation Joint Configurations

Articulation section (36) of shaft (30) may take a variety of forms. By way of example only, articulation section (36) may be configured in accordance with one or more teachings of U.S. Patent App. No. [ATTORNEY DOCKET NO. END6889USNP], entitled "Articulation Joint Features for Articulating Surgical Device," filed on even date herewith. As another merely illustrative example, articulation section (36) may be configured in accordance with one or more teachings of U.S. Patent App. No. [ATTORNEY DOCKET NO. END6889USNP1], entitled "Articulation Joint Features for Articulating Surgical Device," filed on even date herewith. Furthermore, articulation section may be configured in accordance with the teachings of at least one other of the references cited herein. Various other suitable forms that articulation section (36) may take will be apparent to those of ordinary skill in the art in view of the teachings herein.

### III. Exemplary Articulation Control Configurations

As noted above, some versions of handpiece (20) include an articulation control (28), which is operable to control articulation section (36) of shaft (30) to thereby selectively position end effector (40) at various angles relative to the longitudinal axis defined by sheath (32). Several examples of forms that articulation control (28) and other components of handpiece (20) may take will be described in greater detail below, while further examples will be apparent to those of ordinary skill in the art in view of the teachings herein. By way of example only, some merely illustrative alternative examples of articulation control (28) are disclosed in U.S. Patent App. No. [ATTORNEY DOCKET NO. END6888USNP], entitled "Control Features for Articulating Surgical Device," filed on even date herewith.

### A. Exemplary Articulation Control with Laterally Pivoting Trigger

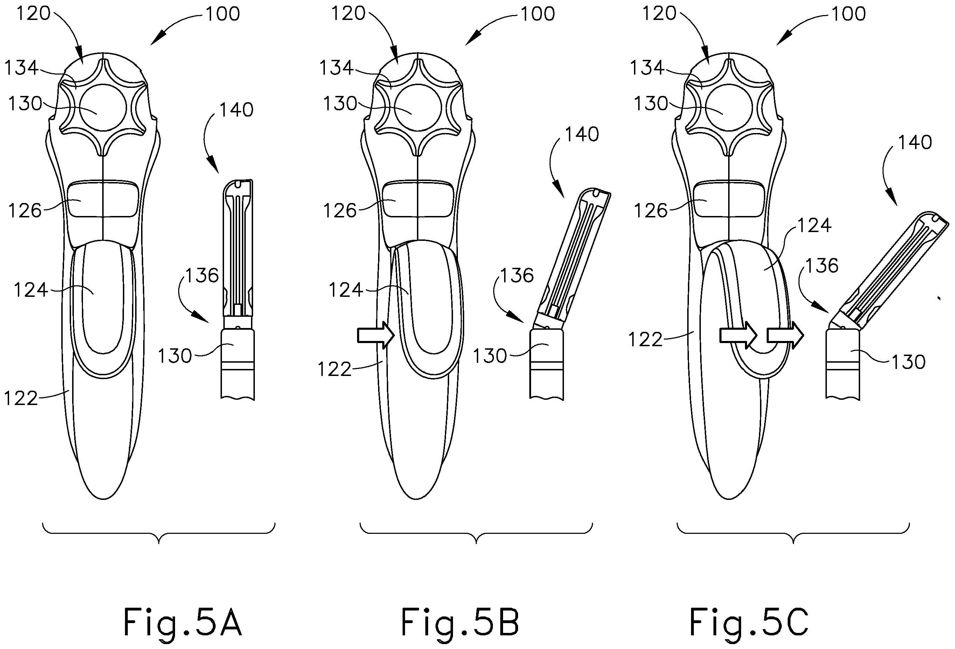

FIGS. 5A-5C show an exemplary electrosurgical instrument (100) that includes a handpiece (120), a shaft (130) extending distally from handpiece (120), and an end effector (140) disposed at a distal end of shaft (130). Handpiece (120) of the present example includes a pistol grip (122), a trigger (124), and an activation button (126). Trigger (124) is pivotable toward and away from pistol grip (122) to selectively actuate end effector (140) as described above and as described in one or more reference cited herein. Activation button (126) is operable to selectively activate RF circuitry that is in communication with end effector (140), as also described above and as described in one or more references cited herein. In some versions, activation button (126) also serves as a mechanical lockout against trigger (124), such that trigger (124) cannot be fully actuated unless button (126) is being pressed simultaneously. Examples of how such a lockout may be provided are disclosed in one or more of the references cited herein. It should be understood that pistol grip (122), trigger (124), and button (126) may be modified, substituted, supplemented, etc. in any suitable way, and that the descriptions of such components herein are merely illustrative. Trigger (124) of the present example is also operable to selectively control articulation joint (136) of shaft (130), as will be described in greater detail below.

Shaft (130) of the present example includes an articulation joint (136) that pivotally couples end effector (140) with shaft (130). Articulation joint (136) of the present example is operable to selectively position end effector (140) at various angles relative to the longitudinal axis defined by shaft (130). Various examples of forms that articulation joint (136) and other components of shaft (130) may take are described in various references cited herein, while further examples will be apparent to those of ordinary skill in the art in view of the teachings herein. Similarly, end effector (140) may be configured in accordance with end effector (40) described above, in accordance with the teachings of various references cited herein, and/or in any other suitable way as will be apparent to those of ordinary skill in the art in view of the teachings herein.

In some versions, shaft (130) is also rotatable about the longitudinal axis defined by shaft (130), relative to handpiece (120), via a knob (134). Such rotation may provide rotation of end effector (140) and shaft (130) unitarily. In some other versions, knob (134) is operable to rotate end effector (140) without rotating any portion of shaft (130) that is proximal of articulation joint (136). As another merely illustrative example, electrosurgical instrument (100) may include one rotation control that provides rotatability of shaft (130) and end effector (140) as a single unit; and another rotation control that provides rotatability of end effector (140) without rotating any portion of shaft (130) that is proximal of joint (136). Other suitable rotation schemes will be apparent to those of ordinary skill in the art in view of the teachings herein. Of course, rotatable features may simply be omitted if desired. In any versions of a device that provide rotation of a shaft (130) and/or end effector (140), a rotation knob (134) and/or shaft (130) and/or end effector (140) may include one or more markings facilitating visual identification of the rotational position. For instance, a user may correlate a marking on a rotation knob (134) with a corresponding marking on a shaft (130) and/or end effector (140) to better understand the orientation of such components with respect to the patient and instrument (100).

As noted above, trigger (124) is operable to selectively articulate end effector (140). In particular, trigger (124) is coupled with end effector (140) such that pivoting trigger (124) laterally about a vertical axis such as the vertical axis defined by pistol grip (122) will pivot end effector (140) at articulation joint (136). Such operation is shown in the series of FIGS. 5A-5C, in which trigger (124) pivots about a vertical axis to cause end effector (140) to pivot at joint (136). It should therefore be understood that trigger (124) is selectively pivotable toward and away from a vertical axis such as the vertical axis defined by pistol grip (122) (to actuate end effector (140)); and also selectively pivotable about a vertical axis such as the vertical axis defined by pistol grip (122) (to articulate end effector (140)).

There are numerous ways in which trigger (124) may be coupled with end effector (140) in order to provide pivoting of end effector (140) at joint (136) in response to pivoting of trigger (124) about a vertical axis. For instance, a pair of elongate members (not shown) may extend from a component of handpiece (120) associated with trigger (124), through shaft (130), to end effector (140). Such elongate members may be translatable in opposite directions, such that end effector (140) pivots at joint (136) when one elongate member advances distally while the other elongate member retracts proximally. In addition or in the alternative, the articulation coupling between trigger (124) and end effector (140) may include one or more ratcheting features, such that the user repeatedly pivots trigger (124) laterally to incrementally articulate end effector (140). In some such versions, trigger (124) is resiliently biased to assume the home position shown in FIG. 5A, such that the user may simply push and release trigger (124) several times to articulate end effector (140) in a ratcheting fashion. A release feature may be included to resiliently bias end effector (140) to the home position shown in FIG. 5A, and may be activated (e.g., by a button, etc.) to release end effector (140) from trigger (124) when the user wishes to straighten end effector (140). It should also be understood that end effector (140) may pivot at joint (136) in the same direction in which trigger (124) pivots about a vertical axis; or end effector (140) may pivot at joint (136) in an opposite direction. Other suitable ways in which trigger (124) may be coupled with end effector (140) for articulation will be apparent to those of ordinary skill in the art in view of the teachings herein.

In some versions, trigger (124) may only pivot about a vertical axis when trigger (124) is pivoted away from pistol grip (122), such that trigger (124) may not pivot about a vertical axis once the user starts to squeeze trigger (124) toward pistol grip (122). Various suitable ways in which such operability may be carried out will be apparent to those of ordinary skill in the art in view of the teachings herein. As another merely illustrative variation, a mode switch or other feature may selectively enable trigger (124) to be either pivotable toward and away from pistol grip (122) or pivotable about a vertical axis, but not both at the same time. Other suitable components, features, configurations, and operabilities for electrosurgical instrument (100) will be apparent to those of ordinary skill in the art in view of the teachings herein.

### B. Exemplary Articulation Control with Opposing Cam Sleeves

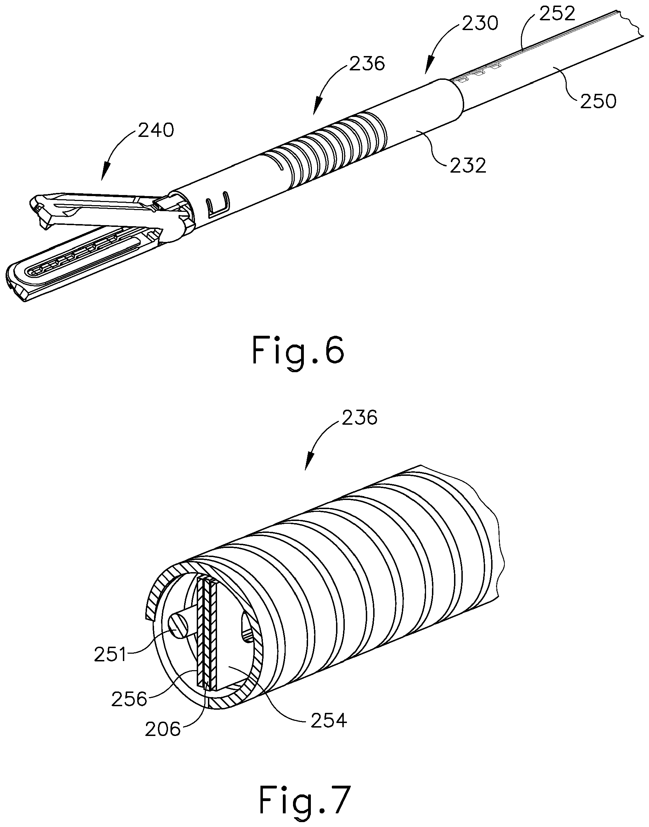

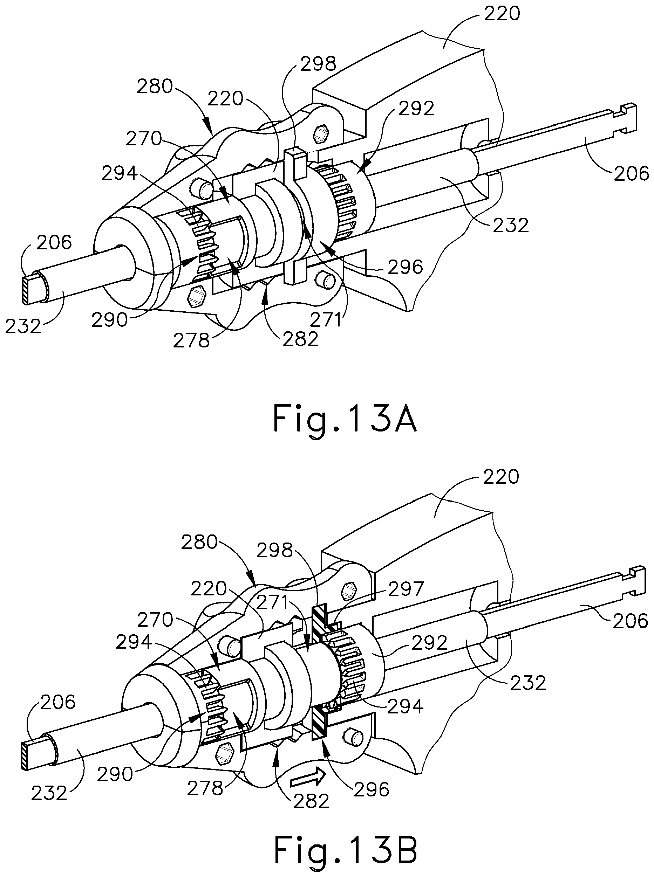

FIGS. 6-15 show additional examples of components that may be used to provide articulation of an end effector (240) at an articulation section (236) of a shaft (230). In this example, shaft (230) extends from a handpiece (220) and includes an outer sheath (232). Articulation section (236) is formed as a helical cutout in sheath (232) in this example, though it should be understood that articulation section (236) may instead be configured in accordance with any other teachings herein and/or the teachings of any suitable reference cited herein. Similarly, while only a portion of handpiece (220) is shown in FIGS. 13A-14B, it should be understood that handpiece (220) may be configured in accordance with any suitable teachings herein and/or the teachings of any suitable reference cited herein. In the present example, a pair of elongate articulation members (250, 252) extend through sheath (232) and are translatable within sheath (232). As will be described in greater detail below, articulation members (250, 252) are operable to translate in opposite directions to selectively bend articulation section (236) away or toward the longitudinal axis defined by shaft (230).

As best seen in FIGS. 7-8, a pair of beams (254, 256) extend through articulation section (236) alongside a firing bar (206). Firing bar (206) is functionally equivalent to firing bar (60) described above. It should therefore be understood that firing bar (206) is translatable within shaft (230) and through articulation section (236) to actuate end effector (240). In the present example, beams (254, 256) are fixedly secured within articulation section (236), such that beams (254, 256) bend with articulation section (236) and such that firing bar (206) translates relative to beams (254, 256). Beams (254, 256) provide structural support to articulation section (236), particularly when articulation section (236) is in a straight configuration; yet beams (254, 256) do not provide undue resistance to bending of articulation section (236) for articulation. Beams (254, 256) also provide lateral support to firing bar (206) to prevent firing bar (206) from buckling or blowing out within articulation section (236), particularly when firing bar (206) is advanced distally under a load while articulation section (236) and firing bar (206) are in a bent configuration. Of course, any other suitable structures or configurations may be used.

As can also be seen in FIG. 7, a wire (251) extends through articulation section (236). Wire (251) also extends through the remainder of shaft (230) and provides electrical communication between end effector (240) and a power source. Wire (251) is flexible such that wire (251) bends with articulation section (236).

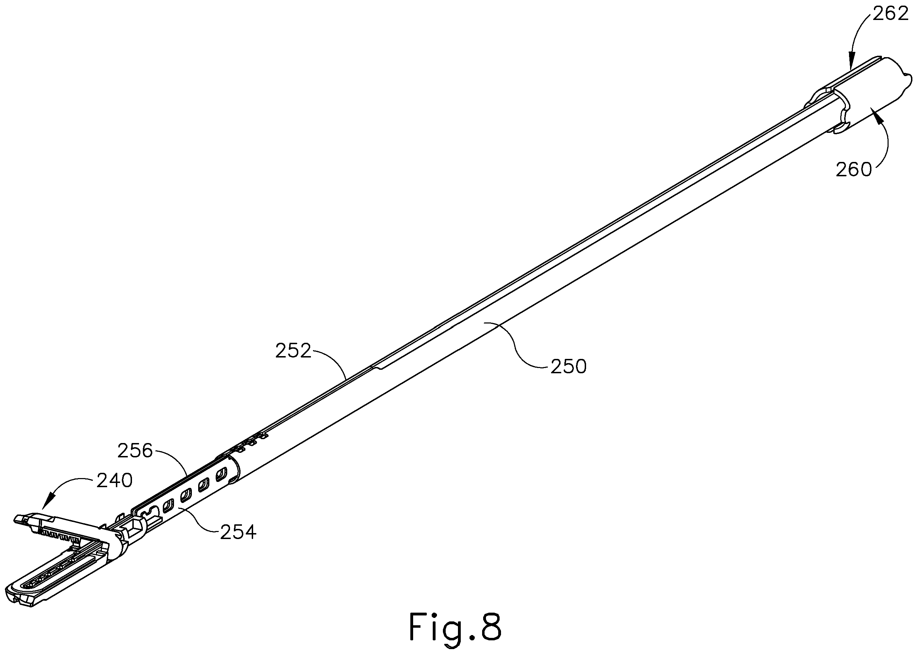

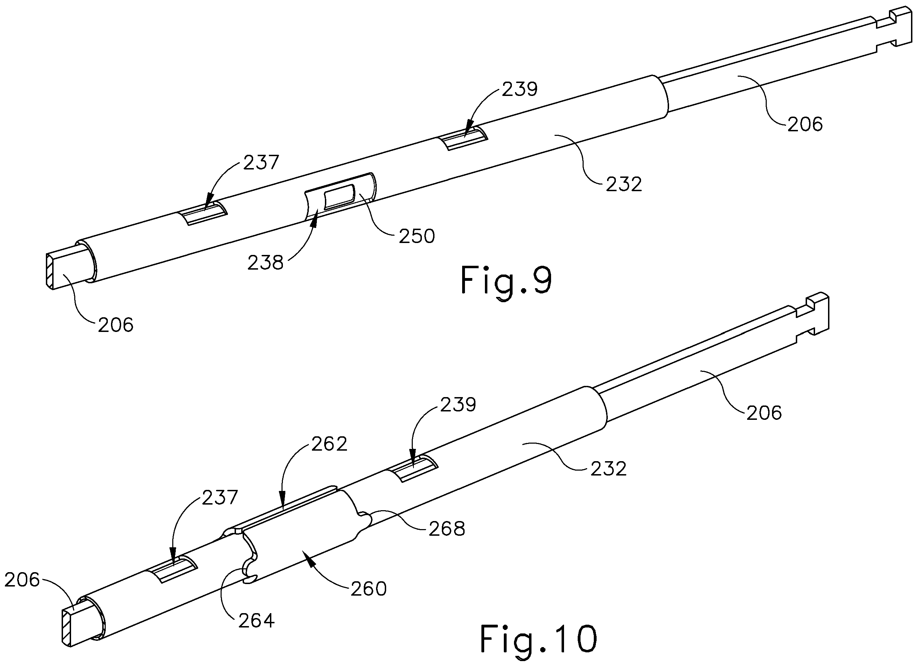

As shown in FIG. 9, sheath (232) includes an opening (238) to reveal a proximal portion of articulation member (250). While not shown, the diametrically opposite side of sheath (232) includes a similar opening to reveal a proximal portion of articulation member (252). As shown in FIGS. 8 and 10-11, a first camming member (260) is fixedly secured to first articulation member (250) through opening (238); while a second camming member (262) is fixedly secured to second articulation member (252) through the other opening. Camming member (260) includes a distal projection (264) and a proximal projection (268). While not shown, camming member (262) includes similar projections. Both camming members (260, 262) are operable to translate longitudinally relative to sheath (232) and relative to each other. In particular, and as will be described in greater detail below, camming members (260, 262) translate in opposing longitudinal directions, to translate articulation members (250, 252) in opposing longitudinal directions relative to sheath (232), to thereby selectively bend articulation section (236). While camming members (260, 262) are translatable through a range of motion relative to sheath (232), camming members (260, 262) will rotate unitarily with sheath (232) in the present example.

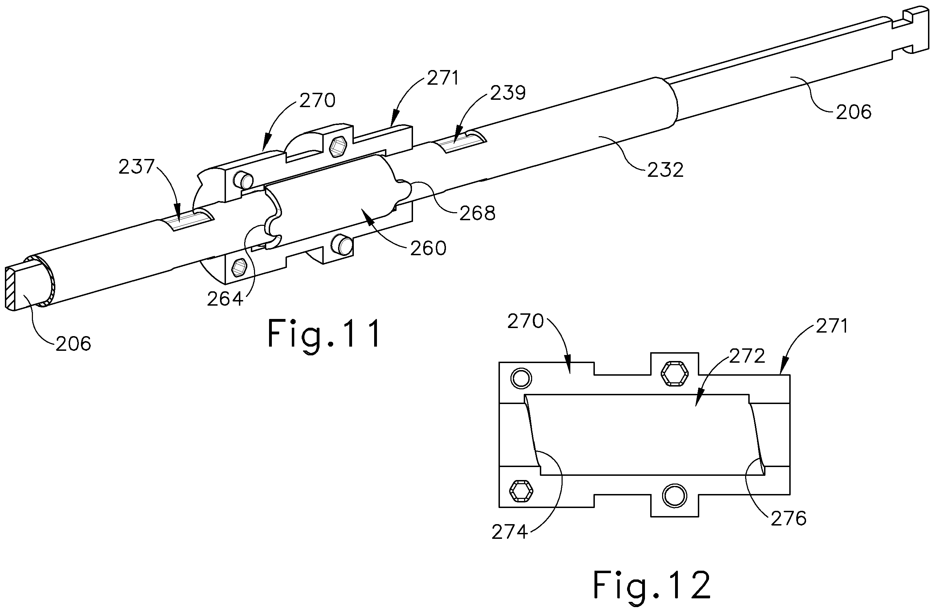

As shown in FIGS. 11-12, a cam nut (270) is coaxially disposed about camming members (260, 262) and about sheath (232). Cam nut (270) defines a hollow interior (272) to accommodate camming members (260, 262) and includes a distal cam surface (274) and a proximal cam surface (276). Cam surfaces (274, 276) are parallel to each other and are oblique relative to the longitudinal axis of sheath (232). Cam surfaces (274, 276) are positioned to maintain contact with projections (264, 268) of camming members (260, 262). Cam nut (270) is rotatable about sheath (232) but does not translate relative to sheath (232). It should be understood that, when cam nut (270) rotates relative to camming members (260, 262) or vice-versa, cam surfaces (274, 276) will bear against projections (264, 268) of camming members (260, 262) to drive camming members (260, 262) longitudinally in opposite directions. Such driving of camming members (260, 262) will correspondingly drive articulation members (250, 252) in longitudinally opposite directions. For instance, rotating cam nut (270) in one direction relative to camming members (260, 262) causes simultaneous distal translation of articulation member (250) and proximal translation of articulation member (252). Rotating cam nut (270) in the opposite direction relative to camming members (260, 262) causes simultaneous proximal translation of articulation member (250) and distal translation of articulation member (252). It should also be understood that such opposing translation of articulation members (250, 252) results in bending of articulation section (236). An example of how components may provide relative rotation between cam nut (270) and camming members (260, 262) will be described in greater detail below, while other examples will be apparent to those of ordinary skill in the art in view of the teachings herein.

As shown in FIGS. 13A-13B, a lock ring (296) is coaxially positioned about a proximal portion (271) of cam nut (270). Lock ring (296) is translatable relative to cam nut (270). While cam nut (270) is rotatable within lock ring (296), the structure of handpiece (220) engages outwardly extending tabs (298) of lock ring (296), thereby preventing lock ring (296) from rotating relative to handpiece (220). As best seen in FIG. 13B, lock ring (296) includes a set of proximally projecting interior splines (297).

As also shown in FIGS. 13A-13B, a distal shaft lock (290) is positioned distal to cam nut (270) while a proximal shaft lock (292) is positioned proximal to cam nut (270). Distal shaft lock (290) is fixedly secured to a distal notch (237) of sheath (232), which is shown in FIGS. 9-11. Proximal shaft lock (292) is fixedly secured to a proximal notch (239) of sheath (232), which is also shown in FIGS. 9-11. Shaft locks (290, 292) are thus both fixed relative to sheath (232). Distal shaft lock (290) includes a set of proximally projecting exterior splines (294). Proximal shaft lock (292) includes a set of distally projecting exterior splines (294). Exterior splines (294) of proximal shaft lock (292) are configured to selectively mesh and lock with interior splines (297) of lock ring (298), as will be described in greater detail below. It should also be understood that the fixed spacing of shaft locks (290, 292) is set such that shaft locks (290, 292) prevent cam nut (270) from translating relative to sheath (232). Furthermore, while shaft locks (290, 292) and cam nut (270) may selectively rotate within handpiece (220) in the present example, the structure of handpiece (220) is configured to prevent shaft locks (290, 292) and cam nut (270) from translating relative to handpiece (220).

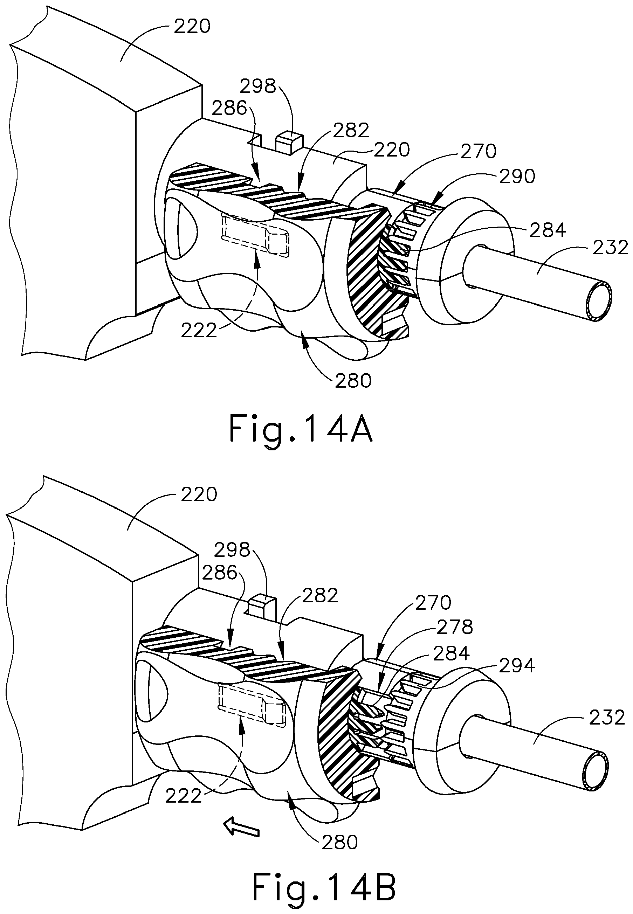

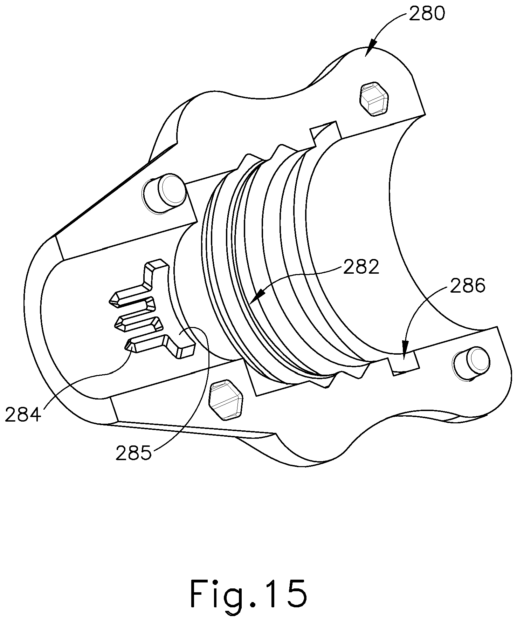

As shown in FIGS. 13A-14B, a rotator (280) is secured at the distal end of handpiece (220). Rotator (280) is rotatable relative to handpiece (220) and is translatable relative to handpiece (220) between a distal position (FIGS. 13A and 14A) and a proximal position (FIGS. 13B and 14B). As best seen in FIG. 15, rotator (280) includes a pair of annular angled recesses (282) and an annular square recess (286). Recesses (282) are configured to selectively engage a resilient detent feature (222) of handpiece (220), to substantially maintain the longitudinal position of rotator (280) relative to handpiece (220). Recess (286) receives tabs (298) of lock ring (296). Recess (286) is sized to permit rotator (280) to rotate freely relative to lock ring (296). However, the relationship between recess (286) and tabs (298) will cause lock ring (296) to translate longitudinally with rotator (280). As will be described in greater detail below, rotator (280) drives interior splines (297) of lock ring (296) into engagement with exterior splines (294) of shaft lock (292) when rotator (280) moved to the proximal position (FIGS. 13B and 14B). Rotator (280) drives interior splines (297) of lock ring (296) out of engagement with exterior splines (294) of shaft lock (292) when rotator (280) is moved to the distal position (FIGS. 13A and 14A).

Rotator (280) also includes a set of distally oriented interior splines (284) extending from a shelf (285). Shelf (285) is positioned within a corresponding recess (278) formed in cam nut (270). Shelf (285) and recess (278) are configured such that shelf (285) remains engaged in recess (278) regardless of whether rotator (280) is in the distal position (FIGS. 13A and 14A) or in the proximal position (FIGS. 13B and 14B). Furthermore, shelf (285) and recess (278) are configured such that their engagement provides unitary rotation of rotator (280) and cam nut (270). In other words, cam nut (270) will rotate with rotator (280) regardless of whether rotator (280) is in the distal position (FIGS. 13A and 14A) or in the proximal position (FIGS. 13B and 14B).

Rotator (280) of the present example is operable to selectively either rotate shaft (230) or bend articulation section (236) to articulate end effector (240), depending on the longitudinal position of rotator (280) relative to handpiece (220) when rotator (280) is rotated relative to handpiece (220). In particular, rotator (280) is operable to rotate shaft (230) relative to handpiece (220), without bending articulation section (236), when rotator (280) is rotated while in the distal position as shown in FIGS. 13A and 14A. Rotator (280) is operable to bend articulation section (236), without rotating shaft (230) relative to handpiece (220), when rotator (280) is rotated while in the proximal position as shown in FIGS. 13B and 14B.

As shown in FIGS 13A and 14A, when rotator (280) is in the distal position, interior splines (284) of rotator (280) are engaged with exterior splines (294) of shaft lock (290). In addition, lock ring (296) is in a distal position when rotator (280) is in the distal position, such that interior splines (297) of lock ring (296) are disengaged from exterior splines (294) of shaft lock (292). Thus, lock ring (296) does not prevent shaft lock (292) or sheath (232) from rotating relative to handpiece (220). With splines (284, 294) engaged and shaft lock (292) being free to rotate relative to handpiece (220), rotation of rotator (280) will cause sheath (232) and end effector (240) to rotate relative to handpiece (220). In addition, since shelf (285) is disposed in recess (278) of cam nut (270), rotation of rotator (280) will also cause cam nut (270) to rotate. Cam nut (270) and sheath (232) thus rotate together with rotator (280) when rotator (280) is in the distal position. Camming members (260, 262) and articulation members (250) also rotate with sheath (232) (and cam nut (270)) due to the relationship between camming members (260, 262) and sheath (232). Since cam nut (270) and camming members (260, 262) rotate together when rotator (280) is in the distal position, articulation section (236) will not bend when rotator (280) is rotated while in the distal position. Thus, when rotator (280) is in the distal position, rotator (280) may be used to rotate the entire shaft (230) and end effector (240) without causing articulation section (236) to bend. It should be understood that such rotation may span an angular range of at least 360°.

As shown in FIGS 13B and 14B, when rotator (280) is in the proximal position, interior splines (284) of rotator (280) are disengaged from exterior splines (294) of shaft lock (290). In addition, lock ring (296) is in a proximal position when rotator (280) is in the proximal position, such that interior splines (297) of lock ring (296) are engaged with exterior splines (294) of shaft lock (292). Thus, lock ring (296) prevents shaft lock (292) from rotating relative to handpiece (220). With the rotational position of shaft lock (292) being secured by lock ring (296), and with splines (284, 290) disengaged, rotation of rotator (280) will not rotate sheath (232) when rotator (280) is in the proximal position. In fact, the relationship between shaft lock (292), lock ring (296), and housing (220) will prevent sheath (232) from rotating at all relative to housing (220) when rotator (280) and lock ring (296) are in the proximal position. Nevertheless, rotator (280) remains free to rotate relative to handpiece (220), and shelf (285) remains engaged with recess (278) of cam nut (270). Thus, rotation of rotator (280) will rotate cam nut (270) relative to handpiece (220). Since the rotational position of sheath (232) is fixed relative to handpiece (220) at this stage, the rotational position of camming members (260, 262) is also fixed at this stage. Thus, cam nut (270) will rotate relative to camming members (260, 262) when rotator (280) is rotated at this stage. As noted above, due to engagement between cam surfaces (274, 276) of cam nut (270) and projections (264, 268) of camming members (260, 262), this relative rotation will cause cam surfaces (274, 276) to bear against projections (264, 268) of camming members (260, 262), thereby driving camming members (260, 262) and articulation members (250, 252) in opposite longitudinal directions. This opposing longitudinal motion of articulation members (250, 252) will cause articulation section (236) to bend, thereby articulating end effector (240). Thus, when rotator (280) is in the proximal position, rotator (280) may be used to bend articulation section (236) without causing shaft (230) or end effector (240) to rotate.

Friction in the articulation control components may substantially prevent articulation section (236) from inadvertently bending during use of end effector (240) when rotator (280) is in the proximal position, such that the articulation control is essentially self locking when placed under a load. When rotator (280) is in the distal position, articulation section (236) will be incapable of bending since the rotational position of cam nut (270) relative to camming members (260, 262) will be fixed. Other suitable ways in which articulation configurations may be selectively held or locked will be apparent to those of ordinary skill in the art in view of the teachings herein.

In some versions, rotator (280) includes a marking providing a visual indication of the articulation angle defined by end effector (240) and shaft (230). Such a marking may be viewed in reference to another marking on handpiece (220). In addition or in the alternative, rotator (280) may include a marking providing a visual indication of the rotational position of end effector (240). Such a marking may also be viewed in reference to another marking on handpiece (220). Of course, these markings are merely optional and may simply be omitted. In some versions, rotator (280) is operable by the same hand that holds handpiece (220) (e.g., by one finger), providing one-handed operation of the entire instrument. As another merely illustrative variation, handpiece (220) may include a mode switch or other type of feature operable to selectively transition rotator (280) between a shaft rotation mode (FIGS. 13A and 14A) and a shaft articulation mode (FIGS. 13B and 14B). Other suitable components, features, configurations, and operabilities will be apparent to those of ordinary skill in the art in view of the teachings herein.

### C. Exemplary Articulation Control with Pivoting Wedge Feature

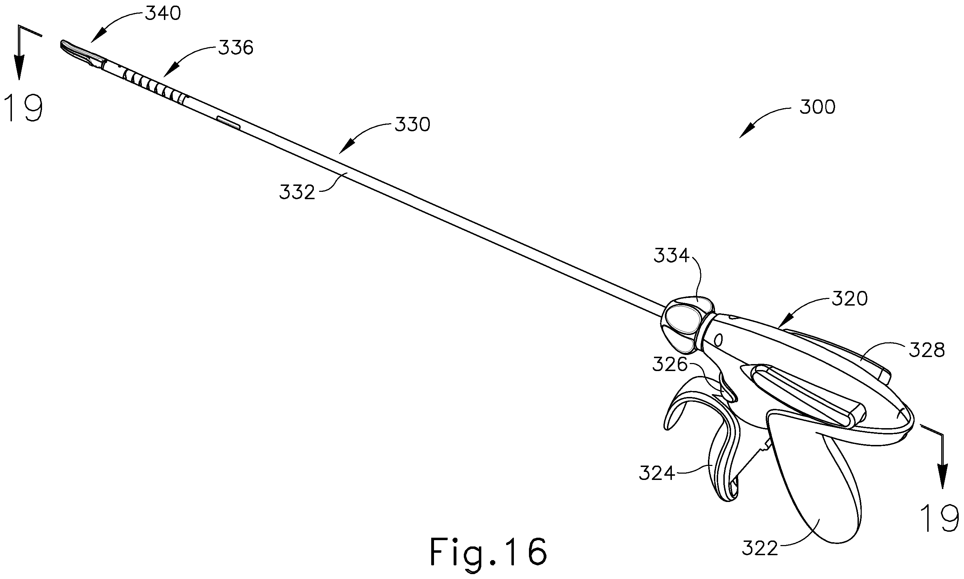

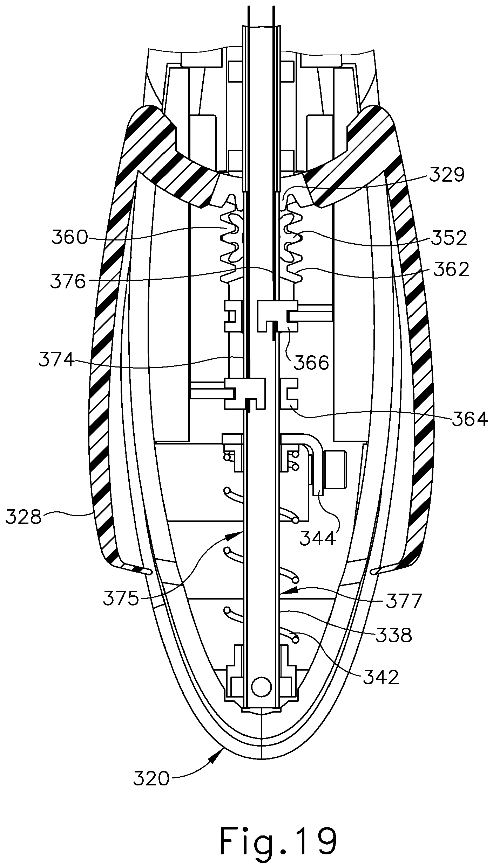

FIGS. 16-19 show another exemplary electrosurgical instrument (300) that includes a handpiece (320), a shaft (330) extending distally from handpiece (320), and an end effector (340) disposed at a distal end of shaft (330). Handpiece (320) of the present example includes a pistol grip (322), a trigger (324), an activation button (326), and an articulation wedge (328). Trigger (324) is pivotable toward and away from pistol grip (322) to selectively actuate end effector (340) as described above and as described in one or more reference cited herein. Activation button (326) is operable to selectively activate RF circuitry that is in communication with end effector (340), as also described above and as described in one or more references cited herein. In some versions, activation button (326) also serves as a mechanical lockout against trigger (324), such that trigger (324) cannot be fully actuated unless button (326) is being pressed simultaneously. Examples of how such a lockout may be provided are disclosed in one or more of the references cited herein. It should be understood that pistol grip (322), trigger (324), and button (326) may be modified, substituted, supplemented, etc. in any suitable way, and that the descriptions of such components herein are merely illustrative. Articulation wedge (328) of the present example is operable to selectively control articulation section (336) of shaft (330), as will be described in greater detail below.

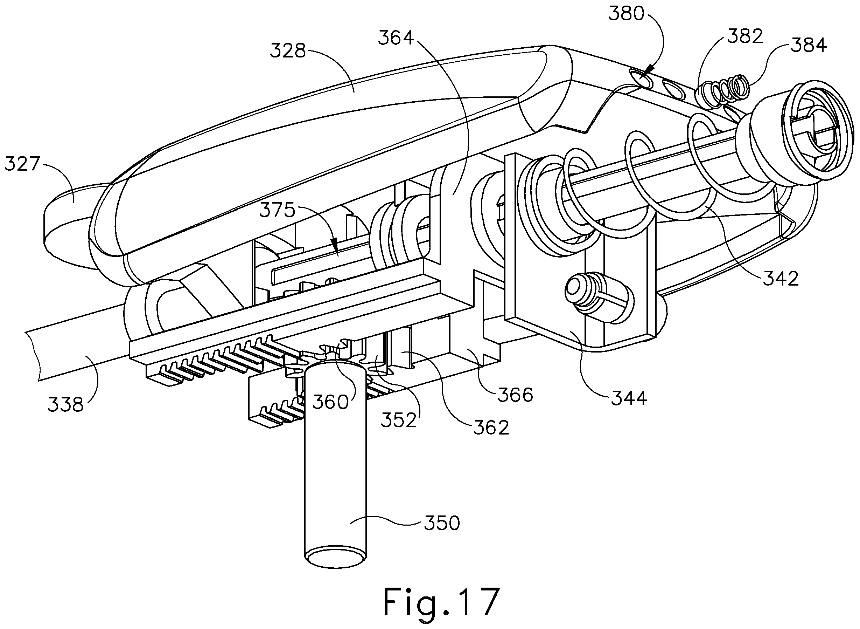

Shaft (330) of the present example includes an outer sheath (332), an articulation section (336) at the distal end of sheath (332), and a cutting member driver tube (338) that is slidably and coaxially disposed within sheath (332). Cutting member driver tube (338) is movable longitudinally to drive a cutting member (not shown) longitudinally. The cutting member is essentially equivalent to firing beam (60) described above. In the present example, driver tube (338) is advanced distally by squeezing trigger (324) toward pistol grip (322); while driver tube (338) is retracted proximally by releasing trigger (324) and/or by actively moving trigger (324) away from pistol grip (322). As shown in FIGS. 17-19, a coil spring (342) is secured to the proximal end of driver tube (338) in the present example. Coil spring (342) is engaged with a bracket (344), which is fixedly secured within handpiece (320). Coil spring (342) thus resiliently biases driver tube (338) to a proximal position. Of course, a driver tube (338) and/or cutting member may be configured and operable in any other suitable fashion.

Articulation section (336) of the present example is operable to selectively position end effector (340) at various angles relative to the longitudinal axis defined by sheath (332). Various examples of forms that articulation section (336) and other components of shaft (330) may take are described in various references cited herein, while further examples will be apparent to those of ordinary skill in the art in view of the teachings herein. Similarly, end effector (340) may be configured in accordance with end effector (40) described above, in accordance with the teachings of various references cited herein, and/or in any other suitable way as will be apparent to those of ordinary skill in the art in view of the teachings herein.

In some versions, shaft (330) is also rotatable about the longitudinal axis defined by sheath (332), relative to handpiece (320), via a knob (334). Such rotation may provide rotation of end effector (340) and shaft (330) unitarily. In some other versions, knob (334) is operable to rotate end effector (340) without rotating any portion of shaft (330) that is proximal of articulation section (336). As another merely illustrative example, electrosurgical instrument (300) may include one rotation control that provides rotatability of shaft (330) and end effector (340) as a single unit; and another rotation control that provides rotatability of end effector (340) without rotating any portion of shaft (330) that is proximal of section (336). Other suitable rotation schemes will be apparent to those of ordinary skill in the art in view of the teachings herein. Of course, rotatable features may simply be omitted if desired. In any versions of a device that provide rotation of a shaft (330) and/or end effector (340), a rotation knob (334) and/or shaft (330) and/or end effector (340) may include one or more markings facilitating visual identification of the rotational position. For instance, a user may correlate a marking on a rotation knob (334) with a corresponding marking on a shaft (330) and/or end effector (340) to better understand the orientation of such components with respect to the patient and instrument (300).

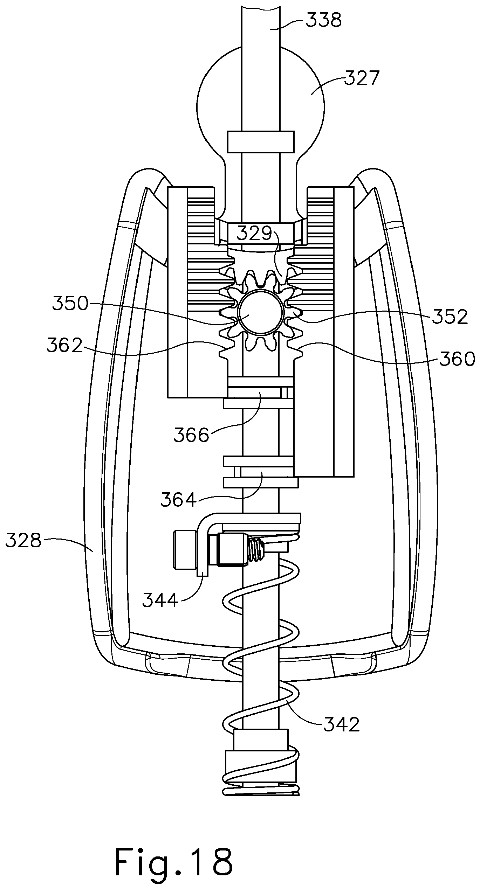

As shown in FIGS. 17-18, a pivot protrusion (329) extends unitarily and distally from articulation wedge (328). Protrusion (329) is movably secured to handpiece (320) such that articulation wedge (328) is pivotable about a vertical pivot axis defined through the center of protrusion (329). This vertical pivot axis is perpendicular to the longitudinal axis defined by shaft (330). As shown in FIGS. 18-19, the underside of articulation wedge (328) includes a proximally presented integral rack (329).

A transmission shaft (350) is positioned beneath articulation wedge (328) and defines a rotation axis that is perpendicular to the axis defined by shaft (330). Shaft (350) is rotatable within handpiece (320). Shaft (350) includes a unitary pinion (352) that meshes with integral rack (329) of articulation wedge (328). Thus, pinion (352) rotates in response to articulation wedge (328) being laterally pivoted/rotated relative to handpiece (320). Pinion (352) is engaged with a pair of opposing racks (360, 362). Rack (360) extends longitudinally within handpiece (320) and is integral with a laterally extending bracket (364); while rack (362) extends longitudinally within handpiece (320) and is integral with a laterally extending bracket (366). As best seen in FIGS. 18-19, brackets (364, 366) are arranged such that they both overlap the longitudinal axis of shaft (330) and such that they are staggered along the length of that longitudinal axis. It should be understood from the foregoing that rotation of pinion (352) will drive racks (360, 362) in opposing longitudinal directions.

Bracket (364) is coupled with an articulation band (374) through a longitudinal slot (375) formed in driver tube (338). Bracket (366) is coupled with an articulation band (376) through a longitudinal slot (377) formed in driver tube (338). Articulation bands (374, 376) extend longitudinally through shaft (330), within driver tube (338). Articulation bands (374, 376) are coupled with articulation section (336) such that moving articulation bands (374, 376) in opposing longitudinal directions will cause articulation section (336) to bend, thereby articulating end effector (340). As noted above, rotation of pinion (352) will drive racks (360, 362) in opposing longitudinal directions. It should therefore be understood that pivoting articulation wedge (328) in either direction relative to handpiece (320) will drive articulation bands (374, 376) in opposing longitudinal directions, thereby bending articulation section (336) to articulate end effector (340). For instance, pivoting articulation wedge (328) in one direction will drive articulation band (374) distally while simultaneously driving articulation band (376) proximally, thereby articulating end effector (340) one way. Pivoting articulation wedge (328) in an opposite direction will drive articulation band (374) proximally while simultaneously driving articulation band (376) distally, thereby articulating end effector (340) the other way.

As shown in FIG. 17, a detent assembly may selectively retain articulation wedge (328) at various pivotal positions. In particular, the proximal edge of articulation wedge (328) includes a series of indentations (380) that are configured to receive a ball (382) that is resiliently urged into indentations (380) by a coil spring (384). Coil spring (384) may be fixedly secured within handpiece (320). Other suitable ways in which the position of articulation wedge (328) may be selectively retained will be apparent to those of ordinary skill in the art in view of the teachings herein. Of course, such retention features may be omitted if desired. It should also be understood that articulation wedge (328) may be operated by the same hand that holds handpiece (320) (e.g., by the thumb and/or by one finger), providing one-handed operation of the entire instrument (300). While several exemplary alternative components, features, configurations, and operabilities for instrument (300) will be described in greater detail below, others will be apparent to those of ordinary skill in the art in view of the teachings herein.

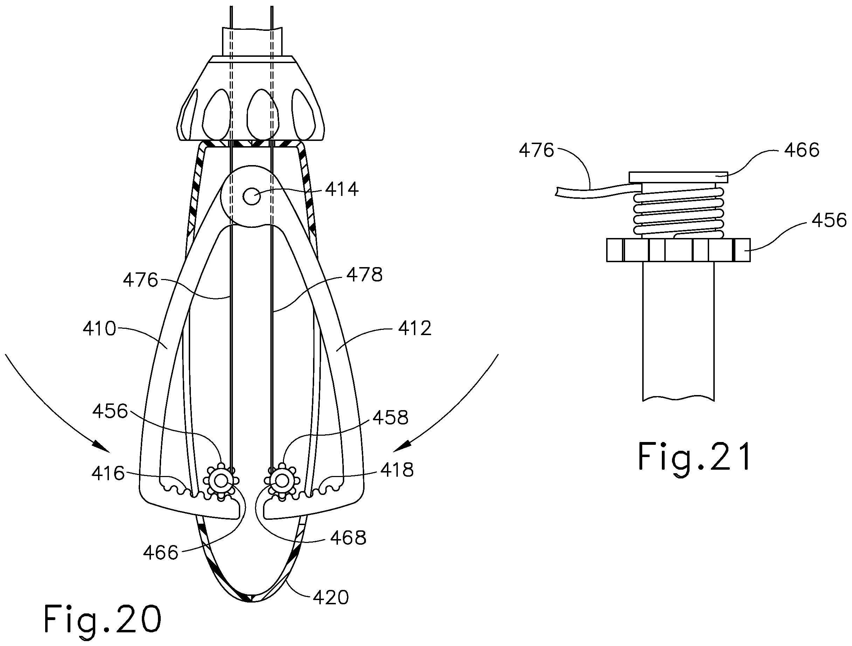

FIG. 20 shows an exemplary alternative to articulation wedge (328) and articulation bands (374, 376). In particular, FIG. 20 shows a pair of articulation arms (410, 412) that are pivotable about a pin (414) secured to a handpiece (420). Arms (410, 412) include respective laterally extending racks (416, 418). Racks (416, 418) mesh with respective pinions (456, 458), which are rotatable within handpiece (420) such that lateral movement of racks (416, 418) will rotate pinions (456, 458). Each pinion (456, 458) is integral with a respective spool (466, 468). A cable (476, 478) is wrapped about each spool (476, 478), such that rotation of spools (466, 468) will wrap or unwrap cables (476, 478) depending on the direction of rotation. Cables (476, 478) are similar to articulation bands (374, 376) in that translation of cables (476, 478) will provide articulation of an end effector. Thus, pivoting articulation arms (410, 412) laterally will translate cables (476, 478) longitudinally, thereby articulating an end effector.

In the present example, articulation arms (410, 412) are movable independently relative to each other. Thus, when arm (410) is pivoted, the effective length of cable (476) will shorten while arm (412) will remain stationary and the effective length of cable (478) will stay the same, resulting in the end effector bending toward arm (410). The opposite result will happen when arm (412) is pivoted. In some other versions, cable (478) is wrapped about spool (458) in an orientation that is opposite to the wrap of cable (476) about spool (456), and articulation arms (410, 412) pivot together in a unitary fashion. Other suitable arrangements will be apparent to those of ordinary skill in the art in view of the teachings herein. It should be understood that the other components, features, configurations, and operabilities of instrument (300) as described above may be readily combined with the components, features, and configurations shown in FIG. 20. It should also be understood that the components, features, and configurations shown in FIG. 20 may be modified, substituted, supplemented, or omitted as desired.

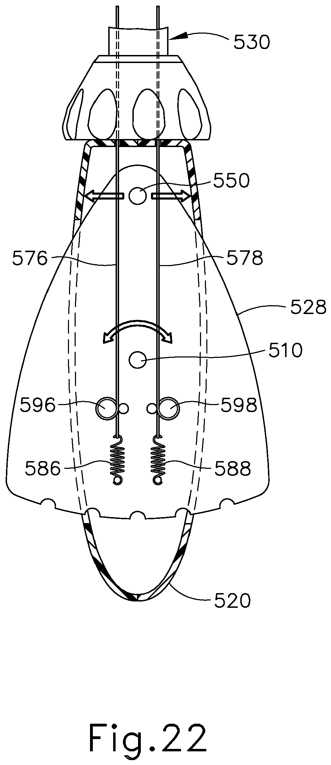

FIG. 22 shows another exemplary alternative to articulation wedge (328) and articulation bands (374, 376). In particular, FIG. 22 shows an articulation wedge (528) that is pivotally secured to a handpiece (520) by a pivot pin (510). A distal post (550) extends upwardly from the distal end of wedge (328). Thus, as wedge (528) pivots about pin (510), post (550) sweeps through an angular range of motion. A pair of cables (576, 578) are secured to handpiece (520) by respective springs (586, 588), which keep cables (576, 578) in tension. Cables (576, 578) are similar to articulation bands (374, 376) in that translation of cables (476, 478) will provide articulation of an end effector. Cables (576, 578) are positioned above wedge (528) and within the sweep path of post (550). Thus, if wedge (528) is pivoted in one direction, post (550) will engage cable (576) and pull cable (576) through shaft (530), thereby reducing the effective length of cable (576). Such reduction in the effective length of cable (576) will articulate an end effector at the distal end of shaft (530). Likewise, if wedge (528) is pivoted in the opposite direction, post (550) will engage cable (578) and pull cable (578) through shaft (530), thereby reducing the effective length of cable (578). Such reduction in the effective length of cable (578) will articulate an end effector at the distal end of shaft (530) in a direction opposite to that of the previous scenario.

As also shown in FIG. 22, posts (596, 598) extend downwardly from housing (520), above wedge (528). Cable (576) is wrapped one time about post (596) and cable (578) is wrapped one time about post (598). While cables (576, 578) are slidable along posts (596, 598), their engagement with posts (596, 598) keeps the proximal ends of cables (576, 578) aligned and prevents springs (586, 588) from deflecting outwardly when either cable (576) is engaged by post (550). In some other versions, posts (596, 598) are integral with wedge (528) and cooperate with post (550) to adjust the effective lengths of cables (576, 578) when wedge (528) is pivoted about pin (510). In some other versions, post (550) may be omitted in favor of including just posts (596, 598) as integral parts of wedge (528). As another merely illustrative alternative, posts (596, 598) may be omitted in favor of including just post (550). Other suitable arrangements will be apparent to those of ordinary skill in the art in view of the teachings herein. It should be understood that the other components, features, configurations, and operabilities of instrument (300) as described above may be readily combined with the components, features, and configurations shown in FIG. 22. It should also be understood that the components, features, and configurations shown in FIG. 22 may be modified, substituted, supplemented, or omitted as desired.

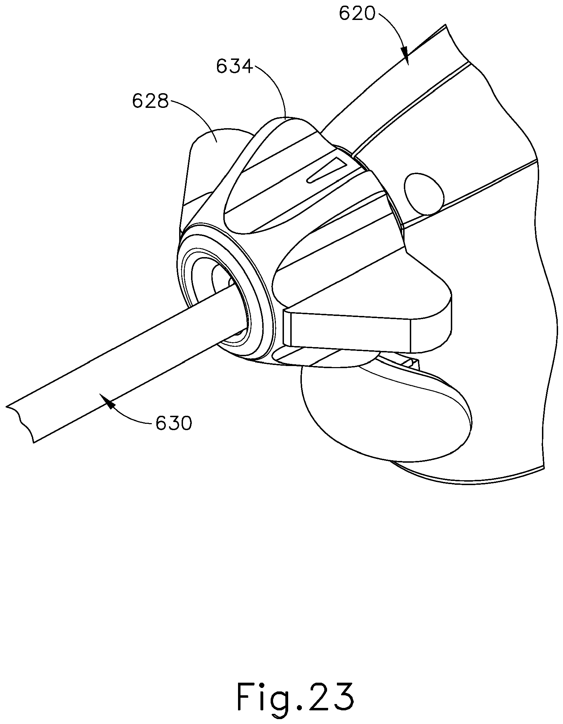

FIG. 23 shows an exemplary articulation wedge (628) positioned within a shaft rotation knob (634). Wedge (628) may be pivoted relative to knob (634) in a manner as described above. Wedge (628) may also be operable to articulate an end effector as described herein. Knob (634) may be rotatable to rotate shaft (630) relative to handpiece (620). Wedge (628) may thus also rotate with knob (634) and shaft (630). It should be understood that the other components, features, configurations, and operabilities of instrument (300) as described above may be readily combined with the components, features, and configurations shown in FIG. 23. It should also be understood that the components, features, and configurations shown in FIG. 23 may be modified, substituted, supplemented, or omitted as desired.

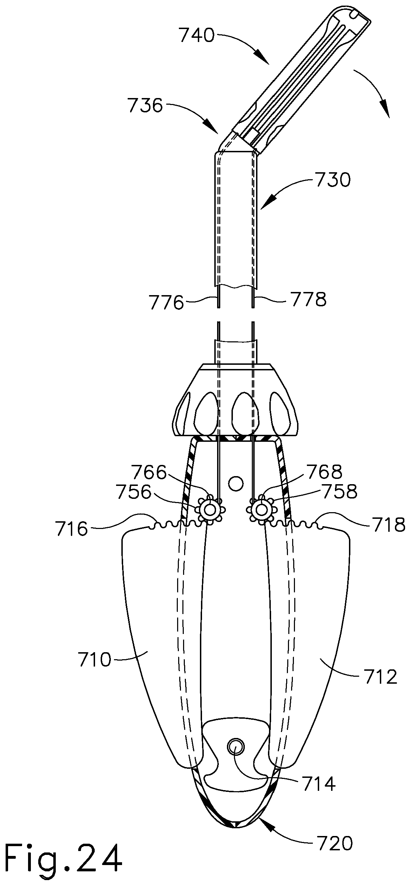

FIG. 24 shows yet another exemplary alternative to articulation wedge (328) and articulation bands (374, 376). In particular, FIG. 24 shows a merely illustrative variation of what is shown in FIG. 20. A pair of articulation arms (710, 712) are pivotable about a pin (714) secured to a handpiece (720). Arms (710, 712) include respective laterally extending racks (716, 718). Racks (716, 718) mesh with respective pinions (756, 758), which are rotatable within handpiece (720) such that lateral movement of racks (716, 718) will rotate pinions (756, 758). Each pinion (756, 758) is integral with a respective spool (766, 768). A cable (776, 778) is wrapped about each spool (766, 768), such that rotation of spools (766, 768) will wrap or unwrap cables (776, 778) depending on the direction of rotation. Cables (776, 778) are similar to articulation bands (774, 776) in that translation of cables (476, 478) through shaft (730) will provide articulation of an end effector (740) at an articulation joint (736). Thus, pivoting articulation arms (710, 712) laterally will translate cables (776, 778) longitudinally, thereby articulating end effector (740) at articulation joint (736). It should be understood that the other components, features, configurations, and operabilities of instrument (300) as described above may be readily combined with the components, features, and configurations shown in FIG. 24. It should also be understood that the components, features, and configurations shown in FIG. 24 may be modified, substituted, supplemented, or omitted as desired.

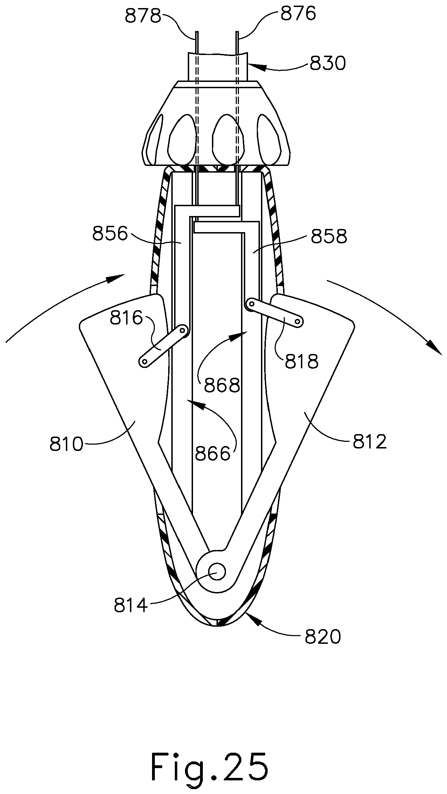

FIG. 25 shows still another exemplary alternative to articulation wedge (328) and articulation bands (374, 376). In particular, FIG. 24 shows a pair of articulation arms (810, 812) that are pivotable about a pin (814) secured to a handpiece (820). A linkage (816) is pivotally coupled to arm (810) and is also pivotally coupled to a bracket (856). Bracket (856) translates longitudinally within a channel (866) defined in handpiece (820). Bracket (856) is also secured to an articulation band (876), which extends through shaft (830) to articulate an end effector at the distal end of shaft (830). It should be understood that the effective length of articulation band (876) in shaft (830) will change based on longitudinal movement of bracket (856) within channel (866). It should also be understood that linkage (816) will convert pivotal movement of arm (810) about pin (814) into longitudinal movement of bracket (856) within channel (866). For instance, if arm (810) is pressed inwardly toward handpiece, (820), bracket (856) will be driven distally, thereby driving articulation band (876) distally.

A linkage (818) is pivotally coupled to arm (812) and is also pivotally coupled to a bracket (858). Bracket (858) translates longitudinally within a channel (868) defined in handpiece (820). Bracket (858) is also secured to an articulation band (878), which extends through shaft (830) to articulate an end effector at the distal end of shaft (830). It should be understood that the effective length of articulation band (878) in shaft (830) will change based on longitudinal movement of bracket (858) within channel (868). It should also be understood that linkage (818) will convert pivotal movement of arm (812) about pin (814) into longitudinal movement of bracket (858) within channel (868). For instance, if arm (812) is pressed inwardly toward handpiece, (820), bracket (858) will be driven distally, thereby driving articulation band (878) distally.

Articulation bands (876, 878) of the present example are similar to articulation bands (374, 376) in that translation of cables (876, 878) will provide articulation of an end effector. It should therefore be understood that pivoting of arm (810) or arm (812) about pin (814) will articulate the end effector. In some versions, when one arm (810, 812) is pivoted, the other arm (810, 812) remains substantially stationary. In some other versions, one arm (810, 812) responsively pivots when the other arm (810, 812) is actively pivoted by a user. For instance, one bracket (856, 858) may responsively translate proximally when the other bracket (856, 858) translates distally. Other suitable arrangements will be apparent to those of ordinary skill in the art in view of the teachings herein. It should be understood that the other components, features, configurations, and operabilities of instrument (300) as described above may be readily combined with the components, features, and configurations shown in FIG. 25. It should also be understood that the components, features, and configurations shown in FIG. 25 may be modified, substituted, supplemented, or omitted as desired.

The examples shown in FIGS. 16-23 include wedge-like articulation control features that define an angle opening proximally, with a vertex positioned at a distal region of a handpiece. Such control features may be best engaged by a user's thumb of a hand grasping a pistol grip of an instrument. FIGS. 24-25 show wedge-like articulation control features that define an angle opening distally, with a vertex positioned at a proximal region of a handpiece. Such control features may be best engaged by a user's index finger of a hand grasping a pistol grip of an instrument. Of course, the articulation control features of FIGS. 16-25 may be engaged in any suitable fashion as desired by the user. Other suitable ways in which the components, features, and configurations of FIGS. 16-23 may be provided and operable will be apparent to those of ordinary skill in the art in view of the teachings herein.

### D. Exemplary Articulation Control with Handpiece Housing

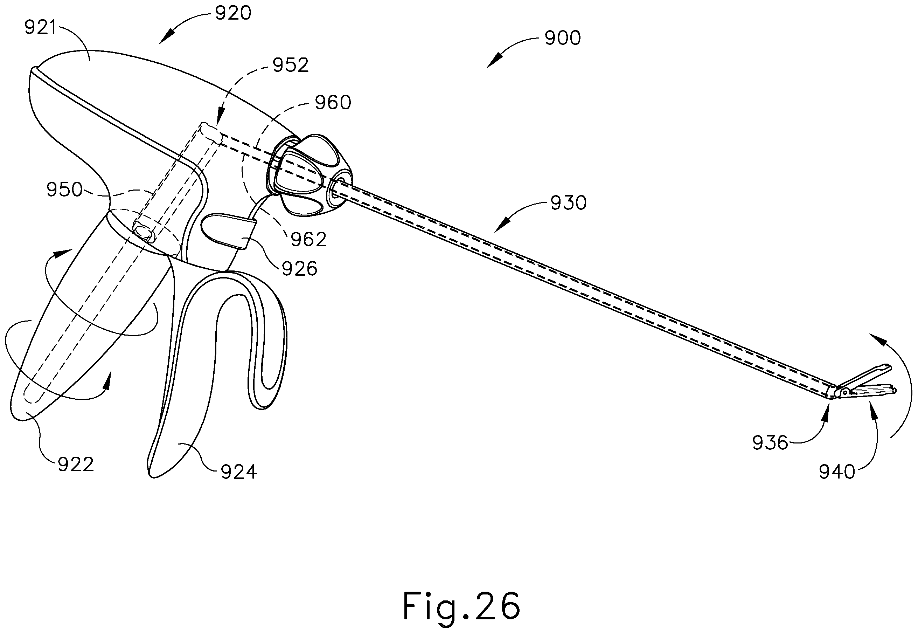

FIG. 26 shows an exemplary electrosurgical instrument (900) that includes a handpiece (920) a shaft (930) extending distally from handpiece (920), and an end effector (940) disposed at a distal end of shaft (930). Handpiece (920) of the present example includes a pistol grip (922), a trigger (924), and an activation button (926). With the exception of pistol grip (922) as described in greater detail below, the above-described components may be configured and operable in accordance with various teachings of such components herein.

Pistol grip (922) of the present example is rotatable relative to housing (921) of handpiece (920). In particular, pistol grip (922) is rotatable about a vertical axis that passes through the center of pistol grip (922) and that is perpendicular to the longitudinal axis defined by shaft (930). A shaft (950) extends upwardly from pistol grip (922), into housing (921), and includes a yoke (952) that is coupled with a pair of cables (960, 962). Cables (960, 962) extend through shaft (930) and are coupled with end effector (940). End effector (940) is pivotally coupled with shaft (930) at an articulation joint (936). Articulation joint (936) of the present example is operable to selectively position end effector (940) at various angles relative to the longitudinal axis defined by shaft (930), based on opposing longitudinal movement of cables (960, 962). It should be understood that the relationship between cables (960, 962) and yoke (952) provides opposing longitudinal movement of cables (960, 962) when pistol grip (922) is rotated relative to housing (921). In particular, when pistol grip (922) is rotated in one direction, cable (960) advances distally while cable (962) retracts proximally, thereby pivoting end effector (940) at articulation joint (936) one way. When pistol grip (922) is rotated in an opposite direction, cable (960) retracts proximally while cable (962) advances distally, thereby pivoting end effector (940) at articulation joint (936) another way.

Of course, yoke (952) and cables (960, 962) may be substituted with a variety of other components, including but not limited to a pinion and opposing racks. Various other suitable components will be apparent to those of ordinary skill in the art in view of the teachings herein. It should also be understood that pistol grip (922) may include a variety of features to facilitate rotation of pistol grip (922) relative to housing (921). By way of example only, pistol grip (922) may include one or more vertical grooves, ribs, knurling, protrusions, elastomeric features, etc.

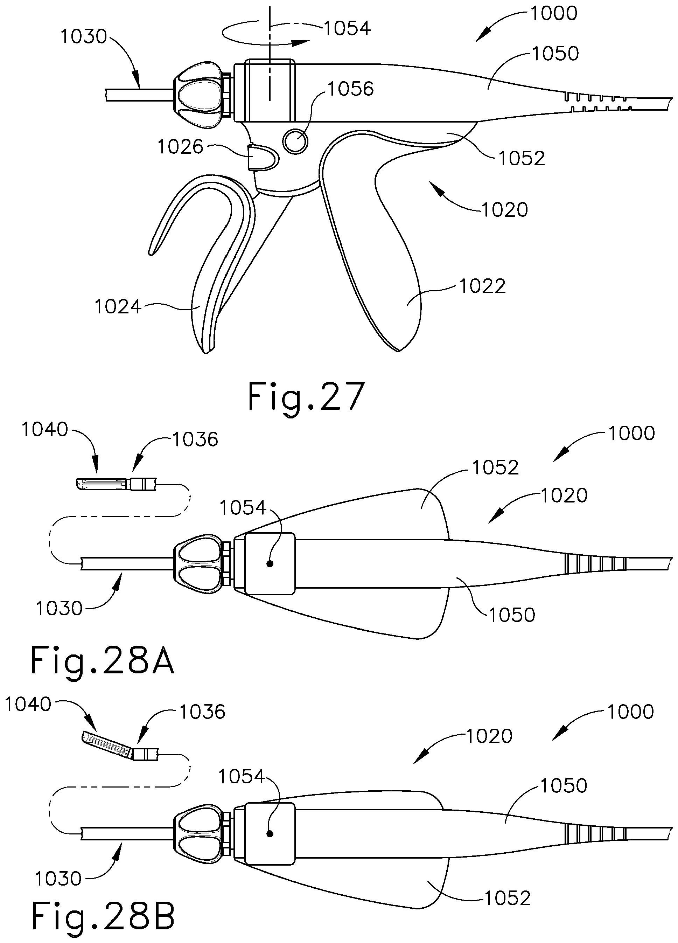

FIGS. 27-28B show an exemplary electrosurgical instrument (1000) that includes a handpiece (1020) a shaft (1030) extending distally from handpiece (1020), and an end effector (1040) disposed at a distal end of shaft (1030). Handpiece (1020) of the present example includes a pistol grip (1022), a trigger (1024), and an activation button (1026). Except as otherwise described below, the above-described components may be configured and operable in accordance with various teachings of such components herein.

Handpiece (1020) of the present example further comprises an upper portion (1050) and a lower portion (1052). As shown, upper portion (1050) is pivotable relative to lower portion (1052) about a pivot axis (1054). As can be seen in the transition from FIG. 28A to FIG. 28B, such pivoting results in corresponding pivoting of end effector (1040) relative to shaft (1030) at an articulation joint (1036). Various components and features that may be used to provide such pivoting of end effector (1040) relative to shaft (1030) in response to pivoting of upper portion (1050) relative to lower portion (1052) will be apparent to those of ordinary skill in the art in view of the teachings herein. It should also be understood that various components and features may be used to keep trigger (1024) sufficiently coupled with end effector (1040) at various angles of articulation.

As shown in FIG. 27, lower portion (1052) includes a release button (1056). Release button (1056) is operable to selectively unlock the position of upper portion (1050) relative to lower portion (1052). For instance, release button (1056) may first be pressed to unlock the position of upper portion (1050) relative to lower portion (1052). While keeping release button (1056) pressed, the user may pivot upper portion (1050) relative to lower portion (1052) or vice versa. In some settings, the user may rely on the position of shaft (1030) in a trocar as a fulcrum or as a mechanical ground to substantially maintain the pivotal position of upper portion (1050) relative to the patient while the user pivots lower portion (1052). Once the user has achieved the desired angle of articulation for end effector (1040), the user may release the release button (1056), thereby locking the pivotal position of portions (1050, 1052) to lock the articulation angle of end effector (1040). Various suitable ways in which release button (1056) may be configured to provide such operability will be apparent to those of ordinary skill in the art in view of the teachings herein. Similarly, various suitable alternatives to release button (1056) will be apparent to those of ordinary skill in the art in view of the teachings herein.

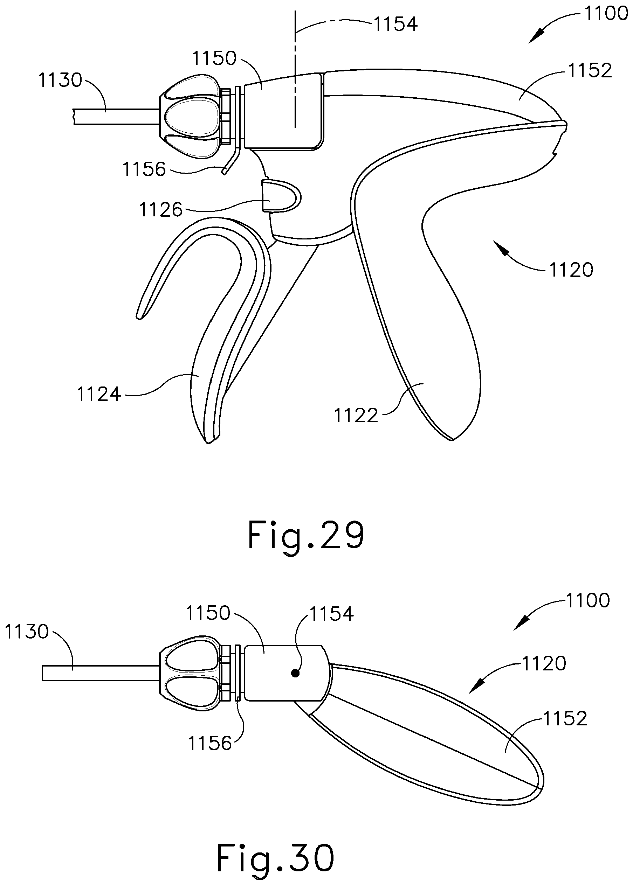

FIGS. 29-30 show an exemplary electrosurgical instrument (1100) that includes a handpiece (1120) and a shaft (1130) extending distally from handpiece (1120). An end effector (not shown) is disposed at a distal end of shaft (1130). Handpiece (1120) of the present example includes a pistol grip (1122), a trigger (1124), and an activation button (1126). Except as otherwise described below, the above-described components may be configured and operable in accordance with various teachings of such components herein.

Handpiece (1120) of the present example further comprises a distal portion (1150) and a proximal portion (1152). As shown, distal portion (1150) is pivotable relative to proximal portion (1152) about a pivot axis (1154). Such pivoting results in corresponding pivoting of an end effector relative to shaft (1130) at an articulation joint (not shown). Various components and features that may be used to provide such pivoting of the end effector relative to shaft (1130) in response to pivoting of distal portion (1150) relative to proximal portion (1152) will be apparent to those of ordinary skill in the art in view of the teachings herein. It should also be understood that various components and features may be used to keep trigger (1124) sufficiently coupled with the end effector at various angles of articulation.

Distal portion (1150) further includes a release trigger (1156). Release trigger (1156) is operable to selectively unlock the position of distal portion (1150) relative to proximal portion (1152). For instance, release trigger (1156) may first be pressed to unlock the position of distal portion (1150) relative to proximal portion (1152). While keeping release trigger (1156) pressed, the user may pivot distal portion (1150) relative to proximal portion (1152) or vice versa. In some settings, the user may rely on the position of shaft (1130) in a trocar as a fulcrum or as a mechanical ground to substantially maintain the pivotal position of distal portion (1150) relative to the patient while the user pivots proximal portion (1152). Once the user has achieved the desired angle of articulation for the end effector, the user may release the release trigger (1156), thereby locking the pivotal position of portions (1150, 1152) to lock the articulation angle of the end effector. Various suitable ways in which release trigger (1156) may be configured to provide such operability will be apparent to those of ordinary skill in the art in view of the teachings herein. Similarly, various suitable alternatives to release trigger (1156) will be apparent to those of ordinary skill in the art in view of the teachings herein.

### E. Exemplary Articulation Control with Clutch

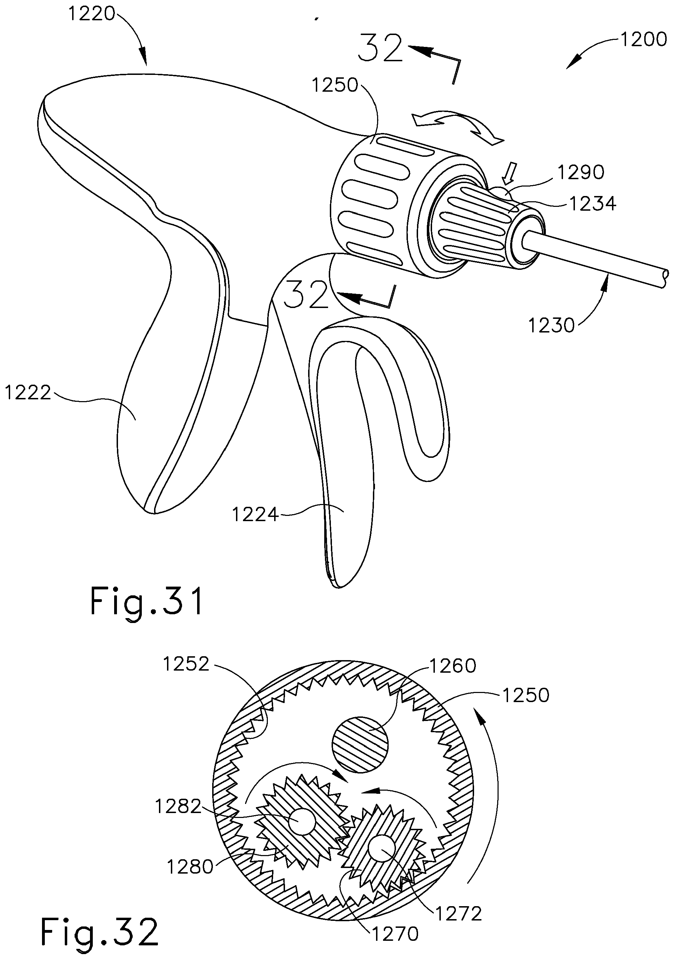

FIGS. 31-35 show an exemplary electrosurgical instrument (1200) that includes a handpiece (1220) and a shaft (1230) extending distally from handpiece (1220). An end effector (not shown) is disposed at a distal end of shaft (1230). Handpiece (1220) of the present example includes a pistol grip (1222), a trigger (1224), and an activation button (not shown). Except as otherwise described below, the above-described components may be configured and operable in accordance with various teachings of such components herein.

Handpiece (1220) of the present example further comprises an articulation dial (1250) and a rotation knob (1234). As shown in FIGS. 32-33, the interior of dial (1250) includes a plurality of teeth (1252). A first gear (1270) meshes directly with teeth (1252). A second gear (1280) meshes directly with first gear (1270) but does not mesh directly with teeth (1252). Thus, when dial (1250) rotates in one direction, first gear (1270) will rotate in the same direction but second gear (1280) will rotate in the opposite direction. A first drive rod (1272) extends through first gear (1270) while a second drive rod (1282) extends through second gear (1280). Rod (1272) and gear (1270) are configured such that rod (1272) rotates with gear (1270) but rod (1272) is translatable relative to gear (1270). Similarly, rod (1282) and gear (1280) are configured such that rod (1282) rotates with gear (1280) but rod (1282) is translatable relative to gear (1280). By way of example only, the portions of rods (1272, 1282) passing through gears (1270, 1280) may have hexagonal cross-sections, with gears (1270, 1280) defining complementary hexagonal openings. As another merely illustrative example, the portions of rods (1272, 1282) passing through gears (1270, 1280) may have keys, with gears (1270, 1280) defining openings having complementary keyways. Other suitable configurations will be apparent to those of ordinary skill in the art in view of the teachings herein. As also shown in FIG. 32, a firing beam (1260) passes through the interior of dial (1250), separate from gears (1270, 1280) and rods (1272, 1282). Firing beam (1260) of this example is essentially equivalent to firing beam (60) described above.

Rotation knob (1234) of the present example is operable to rotate shaft (1230), the components extending through shaft (1230), dial (1250), and gears (1270, 1280) all together, relative to handpiece (1220), and about the longitudinal axis of shaft (1230). Rotation knob (1234) may thus be operable in a manner similar to that described above for rotation knob (34). In the present example, rotation knob (1234) and shaft (1230) remain stationary when dial (1250) rotates, despite the fact that dial (1250) rotates when rotation knob (1234) rotates. Various suitable ways in which such operability may be provided will be apparent to those of ordinary skill in the art in view of the teachings herein.

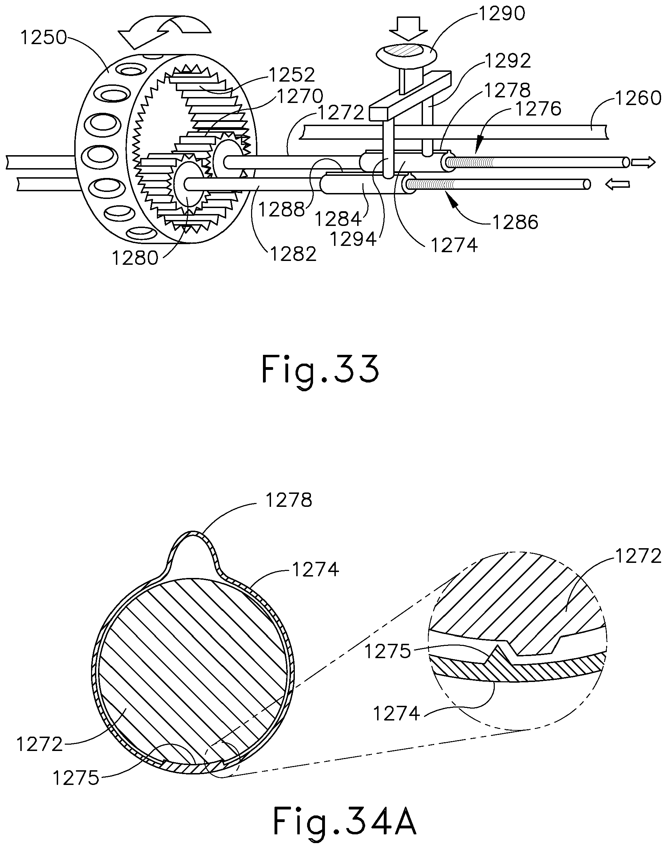

As shown in FIGS. 31 and 33, a clutch button (1290) extends from rotation knob (1234). Clutch button (1290) is operable to simultaneously actuate a pair of stems (1292, 1294). A first stem (1292) is associated with a first cuff (1274); while a second stem (1294) is associated with a second cuff (1284). First cuff (1274) defines internal thread portion (1275) and is disposed about a threaded section (1276) of first rod (1272). First cuff (1274) also defines an upper protrusion (1278) that is engaged by first stem (1292). Second cuff (1284) defines internal thread portion (not shown) and is disposed about a threaded (1288) section of second rod (1282). Second cuff (1284) defines an upper protrusion (1286) that is engaged by second stem (1294). Both cuffs (1274, 1284) are deformable to selectively disengage their respective threaded sections (1276, 1278), as will be described in greater detail below. However, cuffs (1274, 1284) are resiliently biased to engage their internal thread portions with the threaded sections (1276, 1278) of rods (1272, 1282).

When the internal thread portions of cuffs (1274, 1284) are engaged with threaded sections (1276, 1278) of rods (1272, 1282), cuffs (1274, 1284) will act as fixed nuts while threaded sections (1276, 1278) of rods (1272, 1282) act as lead screws. Thus, when rods (1272, 1282) are rotated relative to cuffs (1274, 1284), rods (1272, 1282) will translate. Since rods (1272, 1282) always rotate in opposite directions in this example, and since the thread orientation is the same in both threaded sections (1276, 1278), rods (1272, 1282) will translate in opposite directions when rods (1272, 1282) are rotated relative to cuffs (1274, 1284). Rods (1272, 1282) are similar to articulation bands (374, 376) described above, in that translation of rods (1272, 1282) will provide articulation of an end effector. In particular, translation of rods (1272, 1282) in opposite directions will result in articulation of an end effector relative to shaft (1230). One or more bearings may be positioned at the distal end of rods (1272, 1282) to absorb rotary motion of rods (1272, 1282), such that the distal ends of rods (1272, 1282) only ultimately transfer longitudinal motion for articulation.

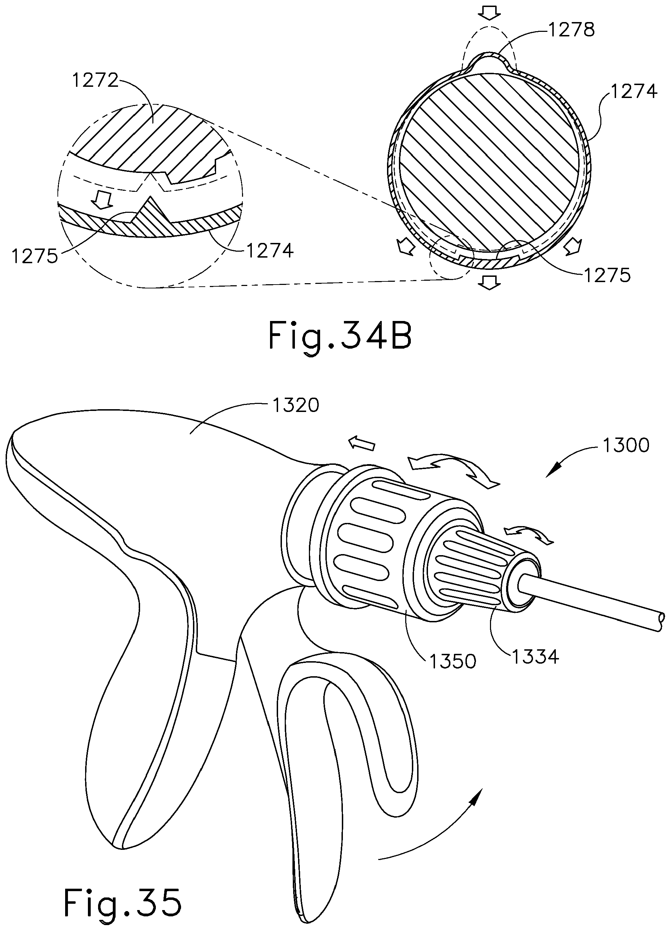

As shown in the transition from FIG. 34A to FIG. 34B, cuffs (1274, 1284) are deformable to selectively disengage their thread portions from threaded sections (1276, 1286) of rods (1270, 1280). Such disengagement allows rods (1270, 1280) to rotate and translate freely relative to cuffs (1274, 1284). Cuffs (1274, 1284) are deformed in response to stems (1292, 1294) pressing downwardly on protrusions (1278, 1288). In some versions, the articulation joint or section of shaft (1230) is resiliently biased to assume a straight configuration. In some such versions, releasing rods (1270, 1280) from cuffs (1274, 1288) will result in sudden and fast return of the articulation joint or section to a substantially straight configuration. Thus, clutch button (1290) provides a release to rapidly straighten out an articulated shaft. Various suitable ways in which an articulation joint or section of shaft (1230) may be resiliently biased to assume a straight configuration will be apparent to those of ordinary skill in the art in view of the teachings herein. It should also be understood that gears (1280, 1270) and/or other components may be configured to resist back-driving, such that an articulated joint or section of shaft (1230) will remain in an articulated configuration during use of instrument (1200) until dial (1250) is rotated or until clutch button (1290) is pressed.

FIG. 35 shows a merely illustrative variation of electrosurgical instrument (1200). In particular, FIG. 35 shows an electrosurgical instrument (1300) having components and operabilities that are substantially identical to those for electrosurgical instrument (1200) described above, except for having a different articulation dial (1350) and a different rotation knob (1334). In this example, rotation knob (1334) lacks an equivalent to clutch button (1290) but is otherwise equivalent to rotation knob (1334). Articulation dial (1350) functions similar to clutch button (1290) when articulation dial (1350) is pulled proximally relative to handpiece (1320). In particular, articulation dial (1350) is operable to disengage cuffs from threaded portions of drive rods, similar to the selective disengagement described above, when articulation dial (1350) is in a proximal position. Articulation dial (1350) is resiliently biased to a distal position, where articulation dial (1350) functions just like articulation dial (1250) described above. Other suitable variations of electrosurgical instruments (1200, 1300) will be apparent to those of ordinary skill in the art in view of the teachings herein.

### F. Exemplary Articulation Control through Multi-Function Trigger

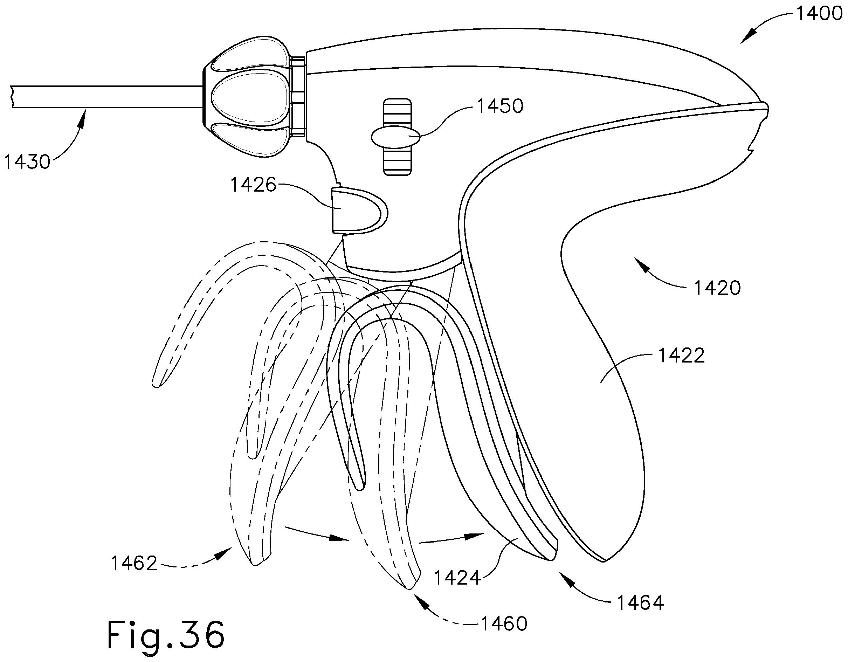

FIG. 36 shows an exemplary electrosurgical instrument (1400) that includes a handpiece (1420) and a shaft (1430) extending distally from handpiece (1420). An end effector (not shown) is disposed at a distal end of shaft (1430). Handpiece (1420) of the present example includes a pistol grip (1422), a trigger (1424), and an activation button (1426). Except as otherwise described below, the above-described components may be configured and operable in accordance with various teachings of such components herein.

In the present example, trigger (1424) is selectively operable either to actuate the end effector in a manner as described above or to articulate the end effector relative to shaft (1430). Handpiece (1420) includes a mode selection toggle switch (1450) enabling the user to select which function will be performed by trigger (1424). When switch (1450) is in one position, trigger (1424) will actuate the end effector in a manner as described above. When switch (1450) is in another position, trigger (1424) will articulate the end effector relative to shaft (1430). If the user uses trigger (1424) to articulate the end effector, the user may move switch (1450) while using trigger (1424) to hold the end effector in the articulated position. Moving switch (1450) at this stage will lock the end effector in the articulated position.

In some versions, when moving switch (1450) is positioned to place trigger (1424) in an articulation control mode, trigger (1424) may be moved from a home position (1460) to a distal position (1462) in order to articulate the end effector in a first direction; or from the home position (1460) to a proximal position (1464) in order to articulate the end effector in a second direction, opposite to the first direction. In some other versions, switch (1450) is movable between three positions - a position where trigger (1424) will actuate the end effector, a position where trigger (1424) will articulate the end effector to the right, and a position where trigger (1424) will articulate the end effector to the left. In these versions, trigger (1424) will articulate the end effector in the selected direction simply when trigger (1424) is squeezed toward pistol grip (1422). Trigger (1424) may also act like a ratchet during articulation, such that trigger (1424) may be squeezed and released repeatedly to articulate the end effector further in either direction. Various suitable components and features that may be used to provide articulation of an end effector in response to positioning of trigger (1424) will be apparent to those of ordinary skill in the art in view of the teachings herein. Similarly, various suitable components and features that may be used to provide selectability of modes of operation for trigger (1424) will be apparent to those of ordinary skill in the art in view of the teachings herein.

### G. Exemplary Articulation Control through Secondary Trigger

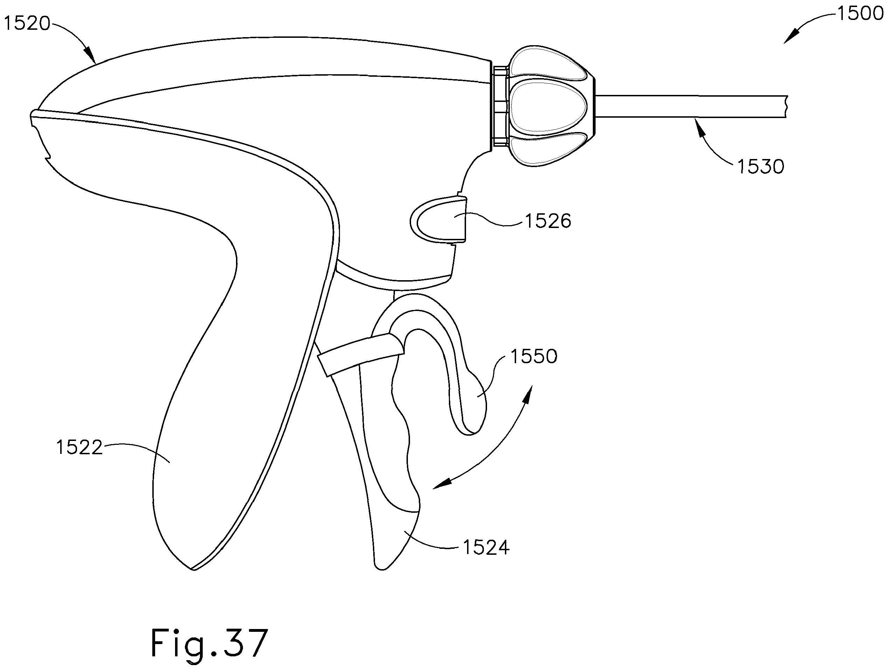

FIGS. 37-38 show an exemplary electrosurgical instrument (1500) that includes a handpiece (1520) and a shaft (1530) extending distally from handpiece (1520). An end effector (not shown) is disposed at a distal end of shaft (1530). Handpiece (1520) of the present example includes a pistol grip (1522), a trigger (1524), and an activation button (1526). Except as otherwise described below, the above-described components may be configured and operable in accordance with various teachings of such components herein.

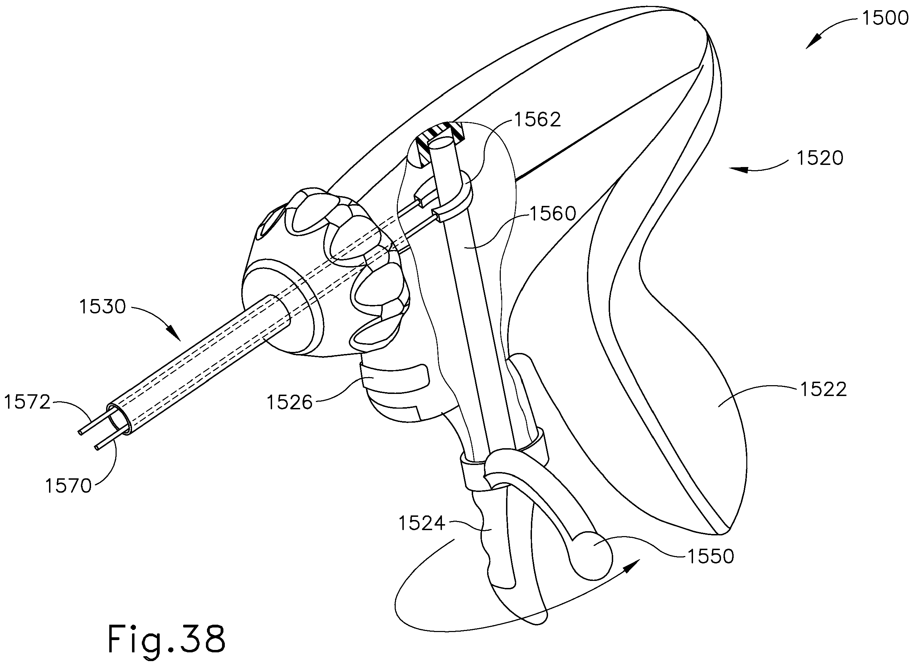

Instrument (1500) of this example includes a secondary lever (1550) that is coupled with trigger (1424). Secondary lever (1550) is also coupled with a shaft (1560), which rotates within handpiece (1520). Secondary lever (1550) thus rotates along a substantially horizontal plane, about a substantially vertical axis, as shown in FIG. 38. In some versions, lever (1550) rotates about an axis defined by trigger (1524). A yoke (1562) is secured to shaft (1560), such that yoke (1562) rotates with shaft (1560). A pair of cables (1570, 1572) are secured to yoke (1562). The relationship between these components provides opposing translation of cables (1570, 1572) in response to rotation of shaft (1560) and yoke (1562). Thus, when secondary lever (1550) is rotated along the substantially horizontal plane, about the substantially vertical axis, cables (1570, 1572) will translate through shaft (1530) in opposing longitudinal directions. Such opposing longitudinal movement of cables (1570, 1572) provides articulation of an end effector at the distal end of shaft (1530), in accordance with various teachings herein. Rotating secondary lever (1550) in one direction along the substantially horizontal plane may articulate end effector one way; while rotating secondary lever (1550) in the opposite direction along the substantially horizontal plane may articulate end effector the other way.

Secondary lever (1550) may also be selectively locked to thereby selectively lock an articulated (or non-articulated) position of the end effector relative to shaft (1530). In particular, secondary lever (1550) is rotatable along a substantially vertical plane, about a substantially horizontal axis, as shown in FIG. 37, to selectively lock. For instance, secondary lever (1550) may be pivoted to an upward position to selectively lock; or to a downward position to selectively unlock. In some versions, secondary lever (1550) may be rotated along the substantially horizontal plane and/or along the substantially vertical plane using just one finger of the hand holding pistol grip (1522), providing complete one-handed operability for instrument (500). Various suitable components, features, and configurations that may be used to provide the above described operability for secondary lever (1550) will be apparent to those of ordinary skill in the art in view of the teachings herein. Similarly, other suitable components, features, configurations, and operabilities for instrument (1500) will be apparent to those of ordinary skill in the art in view of the teachings herein.

### H. Exemplary Articulation Control with Pivoting Cam

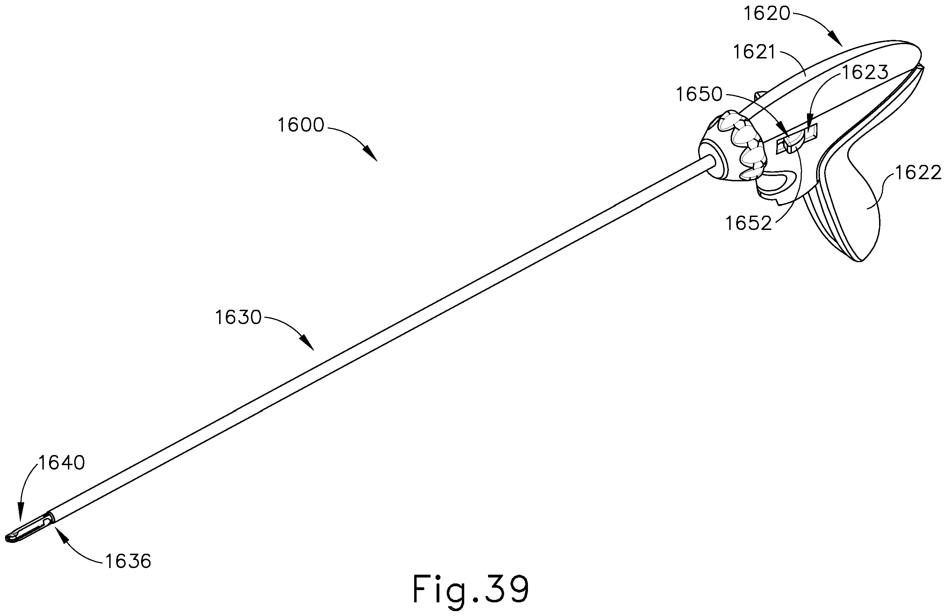

FIGS. 39-43 show an exemplary electrosurgical instrument (1600) that includes a handpiece (1620) and a shaft (1630) extending distally from handpiece (1620). An end effector (1640) is disposed at a distal end of shaft (1630) and articulates relative to shaft (1630) by selectively pivoting at an articulation joint (1636). Handpiece (1620) of the present example includes a pistol grip (1522), a trigger (not shown), and an activation button (not shown). Except as otherwise described below, the above-described components may be configured and operable in accordance with various teachings of such components herein.

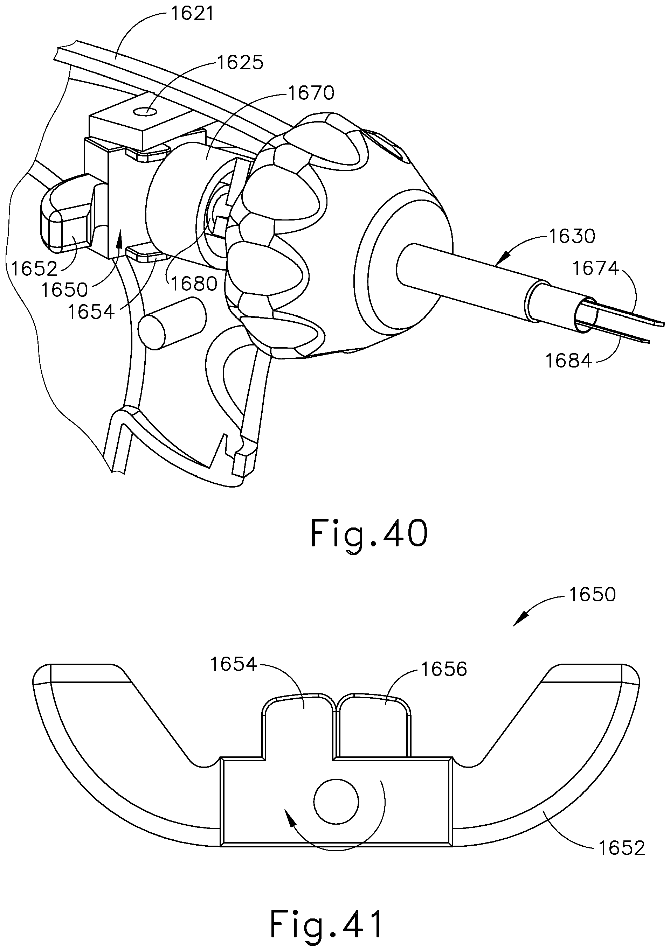

Instrument (1600) of this example includes a cam member (1650) positioned within handpiece (1620). In particular, as shown in FIG. 40, cam member (1650) is pivotally secured within housing (1621) of handpiece (1620) by a pivot pin (1625). As best seen in FIGS. 41-42, cam member (1650) includes a pair of opposing push tabs (1652), a first set of distally extending cams (1654), and a second set of distally extending cams (1656). Referring back to FIG. 39, push tabs (1652) extend outwardly from handpiece (1620), protruding through openings (1623) formed in housing (1621). A user may readily engage push tabs (1652) to selectively pivot cam member (1650) in either direction about pin (1625). Cams (1654) are laterally offset relative to the axis about which cam member (1650) pivots. Cams (1656) are also laterally offset relative to the axis about which cam member (1650) pivots. Thus, when cam member (1650) pivots about pin (1625) in one direction, cams (1654) move distally while cams (1656) move proximally. When cam member (1650) pivots about pin (1625) in the opposite direction, cams (1654) move proximally while cams (1656) move distally.

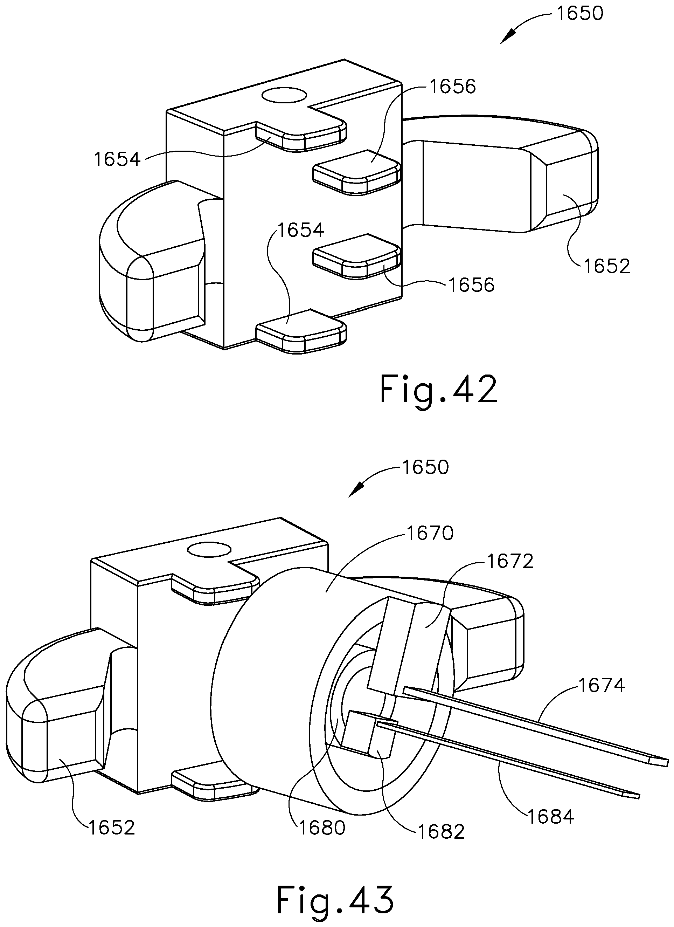

As best seen in FIG. 43, instrument (1600) of this example further includes an outer drive ring (1670) and an inner drive ring (1680). Drive rings (1680) are coaxially positioned and are also located at a common longitudinal position when end effector (1640) is aligned with shaft (1630). Outer drive ring (1670) is in contact with cams (1654) such that outer drive ring (1670) is driven distally by cams (1654) when cam member (1650) pivots in one direction. Inner drive ring (1680) is in contact with cams (1656) such that inner drive ring (1680) is driven distally by cams (1656) when cam member (1650) pivots in the other direction. An arm (1672) extends unitarily and inwardly from outer drive ring (1670) and is further secured to an articulation band (1674). Similarly, an arm (1684) extends unitarily and inwardly from inner drive ring (1680) and is further secured to an articulation band (1684). Thus, articulation bands (1674, 1684) will translate longitudinally with their respective drive rings (1670, 1680). Articulation bands (1674, 1684) extend through shaft (1630) and are operable to pivot end effector (1640) at articulation joint (1636) when bands (1674, 1684) translate in opposite directions. Various suitable ways in which end effector (1640) may pivot in response to opposing longitudinal movement of articulation bands (1674, 1684) will be apparent to those of ordinary skill in the art in view of the teachings herein.

It should be understood from the foregoing that pivoting cam member (1650) about pin (1625) will articulate end effector (1640) based on the direction in which cam member (1650) is pivoted. When cam member (1650) is pivoted in one direction, cams (1654) will push outer drive ring (1670) and bands (1674) distally while cams (1656) will move proximally to allow inner drive ring (1680) and bands (1684) to move proximally. When cam member (1650) is pivoted in the opposite direction, cams (1656) will push inner drive ring (1680) and bands (1684) distally while cams (1654) will move proximally to allow outer drive ring (1670) and bands (1674) to move proximally. Other suitable components, features, configurations, and operabilities that may be provided in instrument (1600) will be apparent to those of ordinary skill in the art in view of the teachings herein.

### IV. Other Exemplary Features

It should be understood that any of the devices herein may also include one or more of the various features disclosed in U.S. Patent App. No. [ATTORNEY DOCKET NO. END6888USNP], entitled "Control Features for Articulating Surgical Device," filed on even date herewith, U.S. Patent App. No. [ATTORNEY DOCKET NO. END6889USNP], entitled "Articulation Joint Features for Articulating Surgical Device," filed on even date herewith, and/or U.S. Patent App. No. [ATTORNEY DOCKET NO. END6889USNP1], entitled "Articulation Joint Features for Articulating Surgical Device," filed on even date herewith.

It should also be understood that any of the devices described herein may be modified to include a motor or other electrically powered device to drive an otherwise manually moved component. Various examples of such modifications are described in U.S. Patent App. No. 13/151,481, entitled "Motor Driven Electrosurgical Device with Mechanical and Electrical Feedback," filed June 2, 1011. Various other suitable ways in which a motor or other electrically powered device may be incorporated into any of the devices herein will be apparent to those of ordinary skill in the art in view of the teachings herein.

It should also be understood that any of the devices described herein may be modified to contain most, if not all, of the required components within the medical device itself. More specifically, the devices described herein may be adapted to use an internal or attachable power source instead of requiring the device to be plugged into an external power source by a cable. Various examples of how medical devices may be adapted to include a portable power source are disclosed in U.S. Provisional Application Serial No. 61/410,603, filed November 5, 2010, entitled "Energy-Based Surgical Instruments." Various other suitable ways in which a power source may be incorporated into any of the devices herein will be apparent to those of ordinary skill in the art in view of the teachings herein.

### VI. Miscellaneous

While the examples herein are described mainly in the context of electrosurgical instruments, it should be understood that the teachings herein may be readily applied to a variety of other types of medical instruments. By way of example only, the teachings herein may be readily applied to tissue graspers, tissue retrieval pouch deploying instruments, surgical staplers, ultrasonic surgical instruments, etc. It should also be understood that the teachings herein may be readily applied to any of the instruments described in any of the references cited herein, such that the teachings herein may be readily combined with the teachings of any of the references cited herein in numerous ways. Other types of instruments into which the teachings herein may be incorporated will be apparent to those of ordinary skill in the art.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Embodiments of the present invention have application in conventional endoscopic and open surgical instrumentation as well as application in robotic-assisted surgery. For instance, those of ordinary skill in the art will recognize that various teaching herein may be readily combined with various teachings of U.S. Pat. No. 6,783,524, entitled "Robotic Surgical Tool with Ultrasound Cauterizing and Cutting Instrument," published August 31, 2004.

Embodiments of the devices disclosed herein can be designed to be disposed of after a single use, or they can be designed to be used multiple times. Embodiments may, in either or both cases, be reconditioned for reuse after at least one use. Reconditioning may include any combination of the steps of disassembly of the device, followed by cleaning or replacement of particular pieces, and subsequent reassembly. In particular, embodiments of the device may be disassembled, and any number of the particular pieces or parts of the device may be selectively replaced or removed in any combination. Upon cleaning and/or replacement of particular parts, embodiments of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, embodiments described herein may be processed before surgery. First, a new or used instrument may be obtained and if necessary cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An electrosurgical device (10), comprising:
(a) a body (20) comprising a handpiece (22), wherein the handpiece defines a first axis;
(b) an end effector (40, 140) comprising:
(i) a first jaw (42), and
(ii) a second jaw (44), wherein the first jaw is movable toward the second jaw to clamp tissue between the first and second jaw,
wherein at least one of the jaws comprises at least one electrode,
wherein the at least one electrode is operable to deliver RF energy to tissue clamped between the first and second jaw;
(c) a cutting member operable to cut tissue clamped between the first jaw and the second jaw;
(d) an actuating member (60) operable to selectively actuate the cutting member; and
(e) a shaft (30) extending between the body and the end effector, wherein the shaft defines a longitudinal axis that is non-parallel to the first axis, wherein the shaft includes an articulation section (136), wherein the articulation section is operable to selectively position the end effector at non-parallel positions relative to the longitudinal axis of the shaft, wherein the body includes a controller (124) operable to selectively actuate the articulation section, wherein the actuating member is in communication with the controller, such that the controller is movable toward and away from the first axis to cause the actuating member to actuate the cutting member and the controller is pivotable about the first axis to selectively actuate the articulation section.

2. The electrosurgical device of claim 1, further comprising a mode selector operable to select an operational mode for the controller, wherein the mode selector is operable to select between a cutting mode and an articulation mode, wherein the controller is operable to cause the actuating member to actuate the cutting member when the mode selector is in the cutting mode, wherein the controller is operable to selectively actuate the articulation section when the mode selector is in the articulation mode.

3. The electrosurgical device of claim 1, further comprising a pair of elongate members extending longitudinally through the shaft, wherein the elongate members are translatable in opposing longitudinal directions to actuate the articulation section, wherein the controller comprises at least one component of the handpiece operable to translate the elongate members in opposing longitudinal directions.

## Patentansprüche

1. Elektrochirurgische Vorrichtung (10), umfassend:
(a) einen Körper (20), der ein Handstück (22) umfasst, wobei das Handstück eine erste Achse definiert,
(b) einen Endeffektor (40, 140), umfassend:
(i) eine erste Backe (42) und
(ii) eine zweite Backe (44),
wobei die erste Backe zu der zweiten Backe hin beweglich ist, um zwischen der ersten und der zweiten Backe Gewebe einzuklemmen,
wobei mindestens eine der Backen mindestens eine Elektrode umfasst,
wobei die mindestens eine Elektrode dahingehend betätigbar ist, HF-Energie an zwischen der ersten und der zweiten Backe eingeklemmtes Gewebe zu liefern,
(c) ein Schneidglied, das dahingehend betätigbar ist, zwischen der ersten und der zweiten Backe eingeklemmtes Gewebe zu schneiden,
(d) ein Betätigungsglied (60), das dahingehend betätigbar ist, das Schneidglied gezielt zu betätigen, und
(e) einen Schaft (30), der sich zwischen dem Körper und dem Endeffektor erstreckt, wobei der Schaft eine Längsachse definiert, die nicht parallel zu der ersten Achse verläuft, wobei der Schaft einen Abwinkelungsabschnitt (136) aufweist, wobei der Abwinkelungsabschnitt dahingehend betätigbar ist, den Endeffektor gezielt in nicht parallelen Positionen bezüglich der Längsachse des Schafts zu positionieren, wobei der Körper eine Steuerung (124) aufweist, die dahingehend betätigbar ist, den Abwinkelungsabschnitt gezielt zu betätigen, wobei das Betätigungsglied mit der Steuerung kommuniziert, so dass die Steuerung zu der ersten Achse hin und von ihr weg beweglich ist, um zu veranlassen, dass das Betätigungsglied das Schneidglied betätigt, und die Steuerung um die erste Achse schwenkbar ist, um den Abwinkelungsabschnitt gezielt zu betätigen.

2. Elektrochirurgische Vorrichtung nach Anspruch 1, ferner umfassend einen Moduswähler, der dahingehend betätigbar ist, einen Betriebsmodus für die Steuerung zu wählen, wobei der Moduswähler dahingehend betätigbar ist, zwischen einem Schneidmodus und einem Abwinkelungsmodus zu wählen, wobei die Steuerung dahingehend betätigbar ist, zu veranlassen, dass das Betätigungsglied das Schneidglied betätigt, wenn der Moduswähler im Schneidmodus ist, wobei die Steuerung dahingehend betätigbar ist, den Abwinkelungsabschnitt gezielt zu betätigen, wenn der Moduswähler im Abwinkelungsmodus ist.

3. Elektrochirurgische Vorrichtung nach Anspruch 1, ferner umfassend ein Paar länglicher Glieder, die sich in Längsrichtung durch den Schaft erstrecken, wobei die länglichen Glieder in entgegengesetzten Längsrichtungen translatierbar sind, um den Abwinkelungsabschnitt zu betätigen, wobei die Steuerung mindestens eine Komponente des Handstücks umfasst, die dahingehend betätigbar ist, die länglichen Glieder in entgegengesetzten Längsrichtungen zu translatieren.

## Revendications

1. Dispositif électrochirurgical (10) comprenant :
(a) un corps (20) comprenant une pièce manuelle (22),
la pièce manuelle définissant un premier axe ;
(b) un effecteur terminal (40, 140) comprenant :
(i) une première mâchoire (42), et
(ii) une seconde mâchoire (44),
la première mâchoire étant mobile vers la seconde mâchoire pour serrer un tissu entre la première mâchoire et la seconde mâchoire,
au moins l'une des mâchoires comprenant au moins une électrode,
la ou les électrodes pouvant fonctionner pour délivrer de l'énergie RF au tissu serré entre la première mâchoire et la seconde mâchoire ;
(c) un élément d'incision actionnable pour couper le tissu serré entre la première mâchoire et la seconde mâchoire ;
(d) un élément d'actionnement (60) actionnable pour actionner de manière sélective l'élément d'incision ;
et
(e) une tige (30) s'étendant entre le corps et l'effecteur terminal, la tige définissant un axe longitudinal qui est non parallèle au premier axe,
la tige comprenant une section d'articulation (136),
la section d'articulation pouvant être actionnée pour positionner de manière sélective l'effecteur terminal à des positions non parallèles par rapport à l'axe longitudinal de la tige, le corps comprenant un dispositif de commande (124) actionnable pour actionner de manière sélective la section d'articulation, l'élément d'actionnement étant en communication avec le dispositif de commande, de telle sorte que le dispositif de commande est mobile vers et à l'opposé du premier axe pour amener l'élément d'actionnement à actionner l'élément d'incision et le dispositif de commande pouvant pivoter autour du premier axe pour actionner de manière sélective la section d'articulation.

2. Dispositif électrochirurgical selon la revendication 1, comprenant en outre un sélecteur de mode actionnable pour sélectionner un mode de fonctionnement pour le dispositif de commande, le sélecteur de mode pouvant fonctionner pour sélectionner entre un mode d'incision et un mode d'articulation, le dispositif de commande étant actionnable pour amener l'élément d'actionnement à actionner l'élément d'incision lorsque le sélecteur de mode est dans le mode d'incision, le dispositif de commande étant utilisable pour actionner de manière sélective la section d'articulation lorsque le sélecteur de mode est dans le mode d'articulation.

3. Dispositif électrochirurgical selon la revendication 1, comprenant en outre une paire d'éléments allongés s'étendant longitudinalement à travers la tige, les éléments allongés pouvant être translatés dans des directions longitudinales opposées pour actionner la section d'articulation, le dispositif de commande comprenant au moins un composant de la pièce manuelle actionnable pour translater les éléments allongés dans des directions longitudinales opposées.
